(19) Europäisches Patentamt
European Patent Office
Office européen des brevets

(11) Veröffentlichungsnummer: **0 109 362**
**A1**

(12) # EUROPÄISCHE PATENTANMELDUNG

(21) Anmeldenummer: **83810518.7**

(22) Anmeldetag: **10.11.83**

(51) Int. Cl.³: **C 07 D 499/00**, A 61 K 31/43 // C07F7/18, C07F9/65, C07D205/08, C07D257/04, C07D271/10

(30) Priorität: **16.11.82 CH 6669/82**

(43) Veröffentlichungstag der Anmeldung: **23.05.84 Patentblatt 84/21**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Lang, Marc, Dr., Rue des Ormes 6, F-68170 Rixheim (FR)**

(54) **Heterocyclylverbindungen, Verfahren zu ihrer Herstellung, pharmazeutische Präparate, welche diese Verbindungen enthalten, und Verwendung von letzteren.**

(57) 2-Heterocyclylniederalkyl-2-penem-Verbindungen der Formel

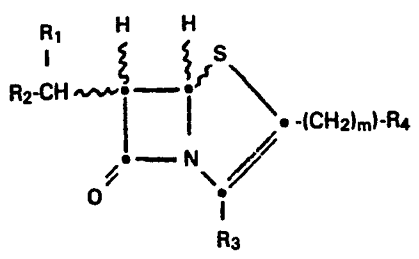

(I),

worin $R_1$ Wasserstoff oder Methyl ist, $R_2$ eine gegebenenfalls geschützte Hydroxyl-Gruppe bedeutet, $R_3$ Carboxyl oder geschütztes Carboxyl $R_3'$ ist, $R_4$ einen über ein Ringkohlenstoffatom an den Rest $-(CH_2)_m-$ gebundenen, ungesättigten monocyclischen Heterocyclyl-Rest darstellt und m eine ganze Zahl von 1 bis 4 ist, Salze von solchen Verbindungen der Formel I, die eine salzbildende Gruppe aufweisen, optische Isomere von Verbindungen der Formel I und Mischungen dieser optischen Isomere besitzen antibiotische Eigenschaften. Die Verbindungen werden nach an sich bekannten Verfahrenn hergestellt.

EP 0 109 362 A1

CIBA-GEIGY AG
Basel (Schweiz)

4-14188/+

Heterocyclylverbindungen, Verfahren zu ihrer Herstellung, pharmazeutische Präparate, welche diese Verbindungen enthalten, und Verwendung von letzteren.

Die vorliegende Erfindung betrifft neue 2-Heterocyclylniederalkylpenem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Heterocyclylniederalkyl-2-penem-Verbindungen der Formel

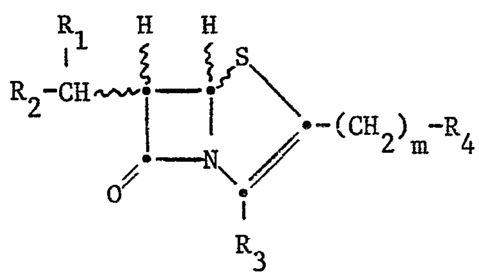

(I),

worin $R_1$ Wasserstoff oder Methyl ist, $R_2$ eine gegebenenfalls geschützte Hydroxyl-Gruppe bedeutet, $R_3$ Carboxyl oder geschütztes Carboxyl $R_3'$ ist, $R_4$ einen über ein Ringkohlenstoffatom an den Rest $-(CH_2)_m-$ gebundenen, ungesättigten, monocyclischen Heterocyclyl-Rest darstellt und m eine ganze Zahl von 1 bis 4 ist, Salze von solchen Verbindungen der Formel I, die eine salzbildende Gruppe aufweisen, optische Isomere von Verbindungen der Formel I und Mischungen dieser optischen Isomere, Verfahren zur Herstellung von Verbindungen der Formel I, pharmazeutische Präparate enthaltend solche Verbindungen und ihre Verwendung zur Herstellung von pharmazeutischen Präparaten oder als pharmakologisch wirksame Verbindungen.

Die vor- und nachstehend verwendeten Definitionen haben im Rahmen der vorliegenden Beschreibung vorzugsweise die folgenden Bedeutungen:

Ein über ein Ringkohlenstoffatom an den Rest $-(CH_2)_m-$ gebundener, ungesättigter monocyclischer Heterocyclyl-Rest $R_4$ ist insbesondere ein entsprechender 5-gliedriger oder 6-gliedriger Heteroaryl-Rest oder partiell gesättigter Heteroaryl-Rest mit 1 bis 4 Ringstickstoffatomen und gegebenenfalls einem zusätzlichen Ringheteroatom der Gruppe Sauerstoff und Schwefel, wie ein entsprechender 5-gliedriger aza-, diaza-, triaza-, tetraza-, oxaza-, oxadiaza-, thiaza-, thiadiaza- oder thiatriaza-cyclischer Rest aromatischen Charakters oder ein entsprechender Dihydro-Rest, oder ein entsprechender 6-gliedriger aza-, diaza- und triaza-cyclischer Rest aromatischen Charakters, sowie ein entsprechenderDihydro-, ferner auch Tetrahydro-Rest. Diese Reste sind unsubstituiert oder können durch gegebenenfalls veräthertes oder verestertes, inklusive geschütztes Hydroxy, z.B. Hydroxy, Niederalkoxy, Niederalkanoyloxy oder Halogen, gegebenenfalls veräthertes Mercapto, z.B. Mercapto, Niederalkylthio oder Phenylthio, Niederalkyl, Hydroxyniederalkyl, Niederalkoxyniederalkyl, Carboxyniederalkyl, gegebenenfalls N-niederalkyliertes Aminoniederalkyl, z.B. Aminoniederalkyl oder Diniederalkylaminoniederalkyl, Sulfoniederalkyl, gegebenenfalls substituiertes, inklusive geschütztes Amino, z.B. Amino, Niederalkylamino, Diniederalkylamino, Niederalkylenamino oder Acylamino, wie Niederalkanoylamino, gegebenenfalls funktionell abgewandeltes, inklusive geschütztes Carboxyl oder Sulfo, z.B. Carboxyl, verestertes Carboxyl, wie Niederalkoxycarbonyl, gegebenenfalls substituiertes Carbamoyl, wie N-mono- oder N,N-diniederalkyliertes Carbamoyl, Cyano, Sulfo oder Sulfamoyl, gegebenenfalls durch Niederalkyl, Nitro, Niederalkoxy und/oder Halogen substituiertes Phenyl, Cycloalkyl, Nitro, Oxo und/oder Oxido substituiert, wie insbesondere mono- oder auch poly-, wie insbesondere disubstituiert, sein.

In der vorliegenden Beschreibung bedeutet der im Zusammenhang mit Definitionen von Gruppen bzw. Verbindungen verwendete Ausdruck

"Nieder", dass die entsprechenden Gruppen bzw. Verbindungen, sofern nicht ausdrücklich anders definiert, bis und mit 7, bevorzugt bis und mit 4 Kohlenstoffatome enthalten.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Niederalkanoyloxy ist z.B. Acetyloxy oder Propionyloxy.

Halogen ist z.B. Fluor, Chlor, Brom oder Jod.

Niederalkylthio ist z.B. Methylthio, Aethylthio, n-Propylthio, Isopropylthio oder n-Butylthio.

Niederalkyl ist z.B. Methyl, Aethyl, n-Propyl, Isopropyl, n-Butyl, Isobutyl, sek.-Butyl oder tert.-Butyl, ferner n-Pentyl, n-Hexyl oder n-Heptyl.

Hydroxyniederalkyl ist z.B. Hydroxymethyl, 2-Hydroxyäthyl oder 2,3-Dihydroxypropyl.

Niederalkoxyniederalkyl ist z.B. Methoxymethyl, 2-Methoxyäthyl, Aethoxymethyl oder 2-Aethoxyäthyl.

Carboxyniederalkyl ist z.B. Carboxymethyl, 1-Carboxy-, 2-Carboxy- oder 1,2-Dicarboxyäthyl.

Aminoniederalkyl ist z.B. Aminomethyl oder 2-Aminoäthyl, während Diniederalkylaminoniederalkyl z.B. Dimethylaminomethyl, 2-Dimethylaminoäthyl oder 2-Diäthylaminoäthyl ist.

Sulfoniederalkyl ist z.B. Sulfomethyl oder 2-Sulfoäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkylenamino weist insbesondere 4 bis 6 Kohlenstoffkettenglieder auf und bedeutet z.B. Pyrrolidino oder Piperidino.

Niederalkanoylamino ist z.B. Acetylamino oder Propionylamino.

Niederalkoxycarbonyl ist z.B. Methoxycarbonyl oder Aethoxycarbonyl.

N-Mono-niederalkyliertes Carbamoyl ist z.B. N-Methyl-, N-Aethyl-, oder N-Propylcarbamoyl, während N,N-di-niederalkyliertes Carbamoyl z.B. N,N-Dimethyl oder N,N-Diäthylcarbamoyl bedeutet.

Cycloalkyl enthält vor allem 3 bis 8, in erster Linie 5 oder 6 Ringglieder und ist z.B. Cyclopentyl oder Cyclohexyl, ferner Cyclopropyl sowie Cycloheptyl.

Entsprechende 5-gliedrige gegebenenfalls partiell gesättigte Heteroarylreste $R_4$ sind z.B. gegebenenfalls durch Niederalkyl substituiertes Pyrrolyl oder Dihydropyrrolyl, z.B. 1-Methyl-2-pyrrolyl oder 4,5-Dihydro-3-pyrrolyl, gegebenenfalls durch Niederalkyl substituiertes Diazolyl, wie Imidazolyl, z.B. 2-Imidazolyl, gegebenenfalls durch Niederalkyl, Carboxyniederalkyl oder Phenyl substituiertes Triazolyl, wie 1H-1,2,3-Triazol-4-yl, 1H-1,2,4-Triazol-3-yl, 1H-1,2,4-Triazol-5-yl oder 4H-1,2,4-Triazol-3-yl, z.B. die entsprechenden unsubstituierten Reste, 1-Methyl-1H-1,2,3-triazol-4-yl, 5-Methyl-1H-1,2,4-triazol-3-yl, 3-Methyl-1-phenyl-1H-1,2,4-triazol-5-yl, 4,5-Dimethyl-, 4-Carboxymethyl- oder 4-Phenyl-4H-1,2,4-triazol-3-yl, gegebenenfalls durch Niederalkyl, Carboxyniederalkyl, Sulfoniederalkyl, Diniederalkylaminoniederalkyl oder gegebenenfalls Substituenten, wie Halogen,

enthaltendes Phenyl substituiertes Tetrazolyl, wie 1H- oder 2H-Tetrazol-5-yl, z.B. 1H-Tetrazol-5-yl, 1-Methyl-1H-tetrazol-5-yl, 1-Carboxymethyl-1H-tetrazol-5-yl, 1-(2-Carboxyäthyl)-1H-tetrazol-5-yl, 1-Sulfomethyl-1H-tetrazol-5-yl, 1-(2-Sulfoäthyl)-1H-tetrazol-5-yl, 1-(2-Dimethylaminoäthyl)-1H-tetrazol-5-yl, 1-Phenyl-1H-tetrazol-5-yl oder 2-Methyl-2H-tetrazol-5-yl, gegebenenfalls durch Niederalkyl oder Amino substituiertes Thiazolyl, wie 2-Thiazolyl, oder Isothiazolyl, wie 3-Isothiazolyl, z.B. 2-Thiazolyl, 4,5-Dimethyl-2-thiazolyl oder 3-Isothiazolyl, gegebenenfalls durch Niederalkyl oder Amino substituiertes Thiadiazolyl, wie 1,2,3-Thiadiazol-4-yl, 1,2,3-Thiadiazol-5-yl, 1,3,4-Thiadiazol-2-yl, 1,2,4-Thiadiazol-3-yl oder 1,2,4-Thiadiazol-5-yl, z.B. die entsprechenden unsubstituierten Gruppen, ferner 2-Methyl-1,3,4-thiadiazol-5-yl, 2-Amino-1,3,4-thiadiazol-5-yl oder 3-Methyl-1,2,4-thiadiazol-5-yl, Thiatriazolyl, z.B. 1,2,3,4-Thiatriazol-5-yl, gegebenenfalls durch Niederalkyl und/oder Phenyl substituiertes Oxazolyl oder Isoxazolyl, wie 2-Oxazolyl, 5-Oxazolyl oder 5-Isoxazolyl, z.B. die entsprechenden unsubstituierten Gruppen, ferner 4-Methyl-5-oxazolyl, 4,5-Diphenyl-2-oxazolyl oder 3-Methyl-5-isoxazolyl, oder gegebenenfalls durch Niederalkyl oder gegebenenfalls durch Nitro substituiertes Phenyl substituiertes Oxadiazolyl, wie 1,2,4-Oxadiazol-5-yl, 1,2,4-Oxadiazol-3-yl oder 1,3,4-Oxadiazol-2-yl, z.B. die entsprechenden unsubstituierten Gruppen, ferner 2-Methyl-1,3,4-oxadiazol-5-yl, 2-Phenyl-1,3,4-oxadiazol-5-yl oder 5-(4-Nitrophenyl)-1,3,4-oxadiazol-2-yl.

Entsprechende 6-gliedrige gegebenenfalls partiell gesättigte Heteroarylreste $R_4$ sind z.B. gegebenenfalls durch Halogen und/oder Oxido substituiertes Pyridyl, wie 2-, 3- oder 4-Pyridyl, z.B. 2-Pyridyl, 3-Pyridyl, 1-Oxido-2-pyridyl oder 4-Chlor-1-oxido-2-pyridyl, gegebenenfalls durch Niederalkyl, Hydroxy, Niederalkoxy, Halogen und/oder Oxido substituiertes Pyridazinyl, wie 6-Pyridazinyl, z.B. 3-Hydroxy-6-pyridazinyl, 2-Oxido-6-pyridazinyl, 3-Chlor-1-oxido-6-pyridazinyl, 3-Methyl-2-oxido-6-pyridazinyl, 3-Methoxy-1-oxido-6-pyridazinyl oder 3-Aethoxy-1-oxido-6-pyridazinyl, gegebenenfalls

durch Niederalkyl, Amino, Diniederalkylamino, Oxo und/oder Carboxy substituiertes 1,2-Dihydropyrimidinyl, wie 1,2-Dihydro-4-pyrimidinyl, z.B. 2-Oxo-1,2-dihydro-4-pyrimidinyl, 6-Methyl-, 5-Methyl-, 6-Amino-, 6-Dimethylamino-, 5-Carboxy- oder 6-Carboxy-2-oxo-1,2-dihydro-4-pyrimidinyl, oder gegebenenfalls durch Niederalkyl und/oder durch bis zu zwei Oxo substituiertes Triazinyl, wie 1,2,4-Triazin-3-yl, insbesondere N-Niederalkyl-5,6-dioxo-1,2,4-triazin-3-yl, z.B. 1-, 2- oder 4-Methyl-5,6-dioxo-1,2,4-triazin-3-yl.

Die in den Verbindungen der Formel I vorhandenen funktionellen Gruppen, wie Hydroxy-, Carboxy-, Amino- oder Sulfogruppen, insbesondere die Hydroxygruppe $R_2$ und die Carboxylgruppe $R_3$, sind gegebenenfalls durch Schutzgruppen geschützt, die in der Penem-, Penicillin-, Cephalosporin- und Peptidchemie verwendet werden.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in

J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe $R_2$, ferner eine im Rest $R_4$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl- oder Trifluoracetyl, gegebenenfallls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist insbesondere die Triniederalkylsilyl-Gruppe.

Eine Carboxylgruppe $R_3$, ferner auch eine im Rest $R_4$ vorhandene Carboxylgruppe, ist üblicherweise in veresterter Form geschützt, wobei die Estergruppe unter schonenden Bedingungen, z.B. unter schwach reduktiven, wie hydrogenolytischen, oder schonend solvolytischen oder insbesondere basisch oder neutral hydrolytischen Bedingungen, leicht spaltbar ist. Eine geschützte Carboxylgruppe kann ferner wie unter physiologischen Bedingungen spaltbare oder leicht in eine andere funktionell abgewandelte Carboxylgruppe, wie in eine andere veresterte Carboxylgruppe umwandelbare, veresterte Carboxylgruppe darstellen.

Solche veresterte Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3

Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls,z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl.

Eine unter physiologischen Bedingungen spaltbare veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet, 1-Niederalkoxyniederalkoxycarbonyl oder auch 1-Niederalkoxycarbonyloxy-niederalkoxycarbonyl, worin Niederalkyl z.B. Methyl, Propyl, Butyl oder insbesondere Aethyl und Niederalkoxy z.B. Methoxy, Aethoxy, Propoxy oder Butoxy ist. Solche Gruppen sind z.B. Niederalkanoyloxymethoxycarbonyl, z.B. Acetoxymethoxycarbonyl oder Pivaloyloxymethoxycarbonyl, Aminoniederalkanoyl-oxymethoxycarbonyl, insbesondere α-Aminoniederalkanoyloxymethoxycarbonyl, z.B. Glycyloxymethoxycarbonyl, L-Valyloxymethoxycarbonyl, L-Leucyloxymethoxycarbonyl, Phthalidyloxycarbonyl, 4-Crotonolactonyl oder 4-Butyrolacton-4-yl, Indanyloxycarbonyl, z.B. 5-Indanyloxycarbonyl, 1-Aethoxycarbonyloxyäthoxycarbonyl, Methoxymethoxycarbonyl oder 1-Methoxyäthoxycarbonyl.

Bevorzugte geschützte Carboxylgruppen $R_3'$ sind die 4-Nitrobenzyloxycarbonyl-, Niederalkenyloxycarbonyl- und die in 2-Stellung durch Niederalkylsulfonyl, Cyano oder Triniederalkylsilyl substituierte Aethoxycarbonylgruppe, sowie unter physiologischen Bedingungen spaltbare veresterte Carboxylgruppen, wie Niederalkanoyloxymethoxycarbonyl und 1-Niederalkoxycarbonyloxy-niederalkoxycarbonyl.

Eine geschützte Aminogruppe kann beispielsweise in Form einer leicht spaltbaren Acylamino-, Acylimino-, verätherten Mercaptoamino-, Silyl- oder Stannylaminogruppe oder als Enamino-, Nitro- oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls, z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl,

wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trissubstituiertes Silyl)-äthoxycarbonyl, wie 2-Triniederalkylsilyläthoxycarbonyl, z.B. 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält.

Entsprechende Silyl- oder Stannylgruppen sind in erster Linie Triniederalkylsilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -äthylhalbesters, und Niederalk-1-en insbesondere 1-Propen bedeutet. Entsprechende Schutzgruppen sind in erster Linie 1-Niederalkanoyl-prop-1-en-2-yl, z.B. 1-Acetyl-prop-1-en-2-yl, oder 1-Niederalkoxy-carbonyl-prop-1-en-2-yl, z.B. 1-Aethoxycarbonyl-prop-1-en-2-yl.

Eine geschützte Sulfogruppe ist in erster Linie eine veresterte, wie mit einem aliphatischen, cycloaliphatischen, cycloaliphatischaliphatischen, aromatischen oder araliphatischen Alkohol, z.B. einem Niederalkanol, oder mit einen Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxylgruppe veräthert sein.

Salze von erfindungsgemässen Verbindungen sind in erster Linie pharmazeutisch verwendbare, nichttoxische Salze von Verbindungen der Formel I. Solche Salze werden beispielsweise von den in Verbindungen der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salzen, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

In den Penemverbindungen der Formel I können die beiden asymmetrischen Kohlenstoffatome in 5- und 6-Stellung in der R-, der S- oder racemischen R,S-Konfiguration vorliegen. Bevorzugt sind die Verbindungen, in denen die Konfiguration des 5-Kohlenstoffatoms derjenigen des natürlichen Penicillins enspricht (5R-Konfiguration). Die Wasserstoffatome in 5- und 6-Stellung können in cis- oder bevorzugt in trans-Stellung zueinander stehen. In der bevorzugten Konfiguration nimmt der Substituent in 6-Stellung die S-Konfiguration ein. Verbindungen der Formel I, worin $R_1$ Methyl ist, besitzen am Kohlenstoffatom 1 der Seitenkette ein weiteres Chiralitätszentrum, das in der racemischen R,S-Konfiguration, der S- oder insbesondere der R-Konfiguration vorliegen kann.

Die Erfindung betrifft insbesondere Verbindungen der Formel I, worin $R_1$ Wasserstoff oder Methyl ist, $R_2$ Hydroxyl oder geschütztes Hydroxyl bedeutet, $R_3$ Carboxyl oder geschütztes Carboxyl $R_3'$, insbesondere unter physiologischen Bedingungen spaltbares verestertes Carboxyl $R_3'$ ist, $R_4$ einen über ein Ringkohlenstoffatom an den Rest $-(CH_2)_m-$ gebundenen 5-gliedrigen oder 6-gliedrigen Heteroaryl-Rest oder partiell gesättigten Heteroaryl-Rest mit 1 bis 4 Ringstickstoffatomen und gegebenenfalls einem zusätzlichen Ringheteroatom der Gruppe Sauerstoff und Schwefel, wie einen entsprechenden 5-gliedrigen aza-, diaza-, triaza-, tetraza-, oxaza-, oxadiaza-, thiaza-, thiadiaza- oder thiatriaza-cyclischen Rest aromatischen Charakters oder einen entsprechenden Dihydro-Rest, oder einen entsprechenden 6-gliedrigen aza-, diaza- oder triaza-cyclischen Rest aromatischen Charakters oder einen entsprechenden Dihydro- oder Tetrahydro-Rest darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Carboxyl, Niederalkoxycarbonyl, gegebenenfalls N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind und m eine ganze Zahl von 1 bis 4 bedeutet, Salze von Verbindungen der Formel I, die eine salzbildende Gruppe aufweisen, optische Isomere von Verbindungen der Formel I und Mischungen dieser optischen Isomere.

Die Erfindung betrifft in erster Linie Verbindungen der Formel I, worin $R_1$ Wasserstoff oder Methyl ist, $R_2$ Hydroxyl bedeutet, $R_3$ Carboxyl, Niederalkanoyloxymethoxycarbonyl, z.B. Pivaloyloxymethoxycarbonyl, oder 1-Niederalkoxycarbonyloxy-niederalkoxycarbonyl, z.B. 1-Aethoxycarbonyloxyäthoxycarbonyl, bedeutet, $R_4$ über ein Ringkohlen-

stoffatom an den Rest $(CH_2)_m$- gebundenes, gegebenenfalls durch Niederalkyl, Carboxyniederalkyl, Sulfoniederalkyl oder Diniederalkylaminoniederalkyl substituiertes Tetrazolyl, wie 1H- oder 2H-Tetrazol-5-yl, z.B. 1H-Tetrazol-5-yl, 1-Methyl-1H-tetrazol-5-yl, 1-Carboxymethyl-1H-tetrazol-5-yl, 1-Sulfomethyl-1H-tetrazol-5-yl, 1-(2-Dimethylaminoäthyl)-1H-tetrazol-5-yl oder 2-Methyl-2H-tetrazol-5-yl, gegebenenfalls durch Niederalkyl oder Amino substituiertes Thiadiazolyl, wie 1,3,4-Thiadiazol-2-yl, z.B. 1,3,4-Thiadiazol-2-yl oder 2-Methyl-1,3,4-thiadiazol-5-yl, gegebenenfalls durch Niederalkyl substituiertes Oxadiazolyl, wie 1,3,4-Oxadiazol-2-yl, z.B. 2-Methyl-1,3,4-oxadiazol-5-yl, oder Pyridyl, z.B. 2-Pyridyl, darstellt und m eine ganze Zahl von 2 bis 4 bedeutet, pharmazeutisch verwendbare Salze von solchen Verbindungen der Formel I, die eine salzbildende Gruppe enthalten, optische Isomere, z.B. das (5R,6S)-Isomere, von Verbindungen der Formel I und Mischungen dieser optischen Isomere.

Die Erfindung betrifft hauptsächlich (5R,6S)-konfigurierte Verbindungen der Formel I, worin $R_1$ Wasserstoff ist, $R_2$ Hydroxyl bedeutet, $R_3$ Carboxyl ist, $R_4$ durch Niederalkyl oder Diniederalkylaminoniederalkyl substituiertes Tetrazol-5-yl, z.B. 1-Methyl-1H-tetrazol-5-yl, 1-(2-Dimethylaminoäthyl)-1H-tetrazol-5-yl oder 2-Methyl-2H-tetrazol-5-yl, oder durch Niederalkyl substituiertes 1,3,4-Oxadiazol-2-yl, z.B. 2-Methyl-1,3,4-oxadiazol-5-yl, darstellt und m 3 ist, und pharmazeutisch verwendbare Salze von Verbindungen der Formel I.

Die Erfindung betrifft vor allem die in den Beispielen genannten Verbindungen der Formel I und deren pharmazeutisch verwendbare Salze.

Die Verbindungen der vorliegenden Erfindung werden nach an sich bekannten Verfahren hergestellt.

Die neuen Verbindungen werden z.B. hergestellt, indem man

a) eine Ylid-Verbindung der Formel

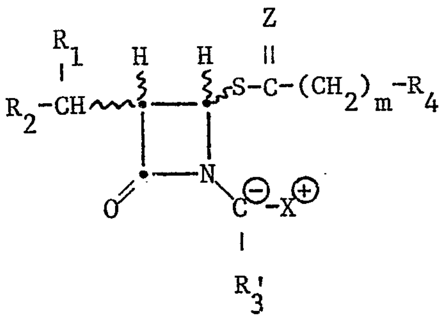

(II),

worin $R_1$, $R_2$, $R'_3$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

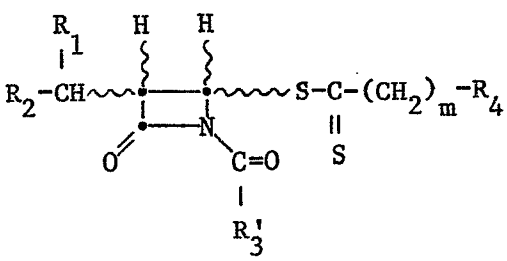

(III) ,

worin $R_1$, $R_2$, $R'_3$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, mit einer organischen Verbindung des dreiwertigen Phosphors behandelt und

wenn erwünscht oder notwendig, in einer erhältlichen Verbindung der Formel I eine geschützte Hydroxylgruppe $R_2$ in die freie Hydroxylgruppe überführt, und/oder, wenn erwünscht, in einer erhältlichen Verbindung der Formel I eine geschützte Carboxylgruppe $R'_3$ in die freie oder in eine andere geschützte Carboxylgruppe $R'_3$ überführt, und/oder, wenn erwünscht, weitere im Rest $R_4$ enthaltende geschützte funktionelle Gruppen in die freien funktionellen Gruppen überführt, und/oder, wenn erwünscht, in einer erhältlichen Verbindung

der Formel I einen Rest $R_4$ in einer anderen Rest $R_4$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

a) Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall-, z.B. Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. Natriumion.

Die Ylid-Verbindungen der Formel II werden in der isomeren Ylen-Form auch als Phosphoran-Verbindungen bezeichnet. In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von

etwa 30° C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Hexan, Cyclohexan, Benzol oder Toluol, einem halogenierten Kohlenwasserstoff, z.B. Methylenchlorid, einem Aether, z.B. Diäthyläther, Dimethoxyäthan oder Diäthylenglykoldimethyläther, einem cyclischen Aether, z.B. Dioxan oder Tetrahydrofuran, einem Carbonsäureamid, z.B. Dimethylformamid, einem Diniederalkylsulfoxid, z.B. Dimethylsulfoxid, oder einem Niederalkanol, z.B. Methanol, Aethanol oder tert.-Butanol, oder in einem Gemisch davon, und, falls notwendig, in einer Inertgas-, z.B. Stickstoffatmosphäre, durchgeführt.

Eine Ausgangsverbindung der Formel II, worin $X^{\oplus}$ eine Phosphonogruppe zusammen mit einem Kation ist, wird vorzugsweise in situ hergestellt, indem man eine Verbindung der Formel

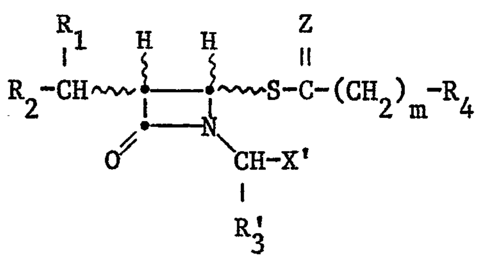

(IIa) ,

worin X' eine Phosphonogruppe bedeutet, mit einem geeigenten basischen Reagenz, wie einer anorganischen Base, z.B. einem Alkalimetallcarbonat, wie Natrium- oder Kaliumcarbonat, oder einer organischen Base, wie einem Triniederalkylamin, z.B. Triäthylamin, oder einer cyclischen Base vom Amidintyp, wie einer entsprechenden Diazabicycloalkenverbindung, z.B. 1,5-Diazabicyclo[5.4.0]undec-5-en, behandelt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/ oder einer gegebenenfalls substituierten aromatischen Hydroxyverbindung, z.B. Phenol oder Brenzcatechin, oder ein Amidester derselben der Formel $P(OR_a)_2-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis etwa 80°C, bevorzugt von etwa 40° bis etwa 60°C, durchgeführt, wobei man 1 Moläquivalent einer Verbindung der Formel III mit 2 Moläquivalenten der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

Die Ausgangsverbindungen der Formel III können beispielsweise hergestellt werden, indem man ein Azetidinon der Formel

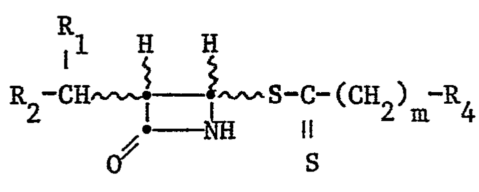

(IV)

mit einer Verbindung der Formel $R_3'-COOH$ oder insbesondere einem reaktionsfähigen Derivat, wie einem Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel III zu Verbindungen

der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

In einer bevorzugten Ausführungsform des Verfahrens stellt man das Ausgangsmaterial der Formel III wie angegeben her und setzt es ohne Isolierung aus dem Reaktionsgemisch mit der organischen Verbindung des dreiwertigen Phosphors um, wobei die Endprodukte der Formel I entstehen.

Bevorzugt werden solche Ausgangsmaterialien der Formeln II und III verwendet, die zu den eingangs als besonders bevorzugt genannten Verbindungen der Formel I führen, insbesondere Verbindungen der Formeln II und III, die eine 4R,3S-Konfiguration aufweisen.

In einer erhältlichen Verbindung der Formel I, worin eine oder mehrere funktionelle Gruppen geschützt sind, können diese, z.B. geschützte Amino-, Carboxyl-, Hydroxy- und/oder Sulfogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino, durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder einem Salz davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel,

oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

In einer verfahrensgemäss erhältlichen Verbindung der Formel I, worin $R_3$ eine geschützte Carboxylgruppe bedeutet und/oder worin der Rest $R_4$ geschütztes Carboxyl als Substituenten enthält, kann die Carboxylgruppe in an sich bekannter Weise freigesetzt werden. So kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzen mit einer Verbindung des Palladiums, z.B.

Tetrakis(triphenylphosphin)palladium, in Gegenwart von Triphenylphosphin und unter Zusatz einer Carbonsäure, z.B. 2-Aethylhexansäure, oder einem Salz davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

In verfahrensgemäss erhältlichen Verbindungen der Formel I, worin $R_2$ eine geschützte Hydroxygruppe darstellt und/oder worin der Rest $R_4$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt, eine Triniederalkylsilylgruppe z.B. mit Tetrabutylammoniumfluorid und Essigsäure (Unter diesen Bedingungen werden durch trisubstituierte Silyläthoxygeschützte Carboxylgruppen nicht gespalten). Eine 2-Halogenniederalkylgruppe und eine gegebenenfalls substituierte Benzylgruppe werden reduktiv abgespalten.

Eine geschützte, insbesondere veresterte, Sulfogruppe wird analog einer geschützten Carboxylgruppe freigesetzt.

Andererseits können auch Verbindungen der Formel I, worin $R_3$ Carboxy, bedeutet, in Verbindungen der Formel I überführt werden, worin $R_3$ eine geschützte Carboxylgruppe, insbesondere eine veresterte Carboxylgruppe, darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig, in Gegenwart einer Lewis-Säure, wie z.B. Bortrifluorid, oder durch Umsetzen mit einem zur Verestertung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloressigsäurechlorid) durch Umsetzen mit Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe kann diese in eine andere veresterte Carboxylgruppe überführt werden, z.B. 2-Chloräthoxycarbonyl oder 2-Bromäthoxycarbonyl durch Behandeln

mit einem Jodsalz, z.B. Natriumjodid, in 2-Jodäthoxycarbonyl. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähigen Ester eines entsprechenden Alkohols in eine Verbindung der Formel I überführen, worin $R_3$ eine unter physiologischen Bedingungen spaltbare veresterte Carboxylgruppe $R_3'$ darstellt.

In Verbindungen der Formel I kann man weiterhin einen Rest $R_4$ in einen anderen Rest $R_4$ überführen.

So kann beispielsweise in Verbindungen der Formel I, worin der Heterocyclyl-Rest $R_4$ durch eine Carboxylgruppe substituiert ist, diese Carboxylgruppe nach an sich bekannten Verfahren in eine funktionell abgewandelte Carboxylgruppe, wie in eine veresterte Carboxylgruppe oder in gegebenenfalls substituiertes Carbamoyl, überführt werden. Beispielsweise erhält man durch Umsetzen einer Verbindung der Formel I, worin $R_4$ durch Carboxyl substituiertes Heterocyclyl ist, mit einem Alkohol, insbesondere einem Niederalkanol, eine Verbindung der Formel I, worin $R_4$ durch verestertes Carboxyl, insbesondere Niederalkoxycarbonyl, substituiertes Heterocyclyl ist, wobei man vorzugsweise in Gegenwart eines geeigenten Kondensationsmittels, z.B. eines Carbodiimids, arbeitet oder das entstehende Wasser durch azeotrope Destillation entfernt. Andererseits kann man Carboxylgruppen an Resten $R_4$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, Ammoniak oder einem primären oder sekundären Amin, z.B. einem Niederalkyl- oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels arbeitet, wie eines aromatischen oder tertiären Amins oder eines Alkalimetall- oder Erdalkalimetallcarbonats.

Weist ein Heteroaryl-Rest $R_4$ eine Hydroxygruppe auf, so lässt sich diese auf üblichem Wege veräthern. Die Umsetzung zu den entsprechenden Niederalkyl-heteroaryläthern erfolgt beispielsweise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden, oder mit Diazoniederalkanen, oder, in Gegenwart eines Dehydatisierungsmittels, beispielsweise Dicyclohexylcarbodiimid, mit Hilfe von Niederalkanolen. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie eines Anhydrids davon, z.B. des symmetrischen Anhydrids davon oder eines gemischten Anhydrids mit einer Halogenwasserstoffsäure, erforderlichenfalls in Gegenwart eines basischen Kondensationsmittels, wie eines Alkalimetallhydroxides oder -carbonates, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierter Hydrolyse, z.B. in Gegenwart von Natriumhydroxid.

In Verbindungen der Formel I, worin $R_4$ durch Amino substituiertes Heterocyclyl bedeutet, kann die Aminogruppe in eine substituierte Aminogruppe, wie eine Niederalkylamino-, Diniederalkylamino-, Niederalkylenamino- oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funktionellen Derivat einer Niederalkancarbonsäure, z.B. dem entsprechenden Carbonsäurehalogenid, in Niederalkanoylamino umgewandelt werden.

Salze von Verbindungen der Formel I mit salzbildenden Gruppen können in an sich bekannter Weise hergestellt werden. So kann man Salze von Verbindungen der Formel I mit einer freien Carboxylgruppe z.B. durch Behandeln mit Metallverbindungen, wie Alkalimetallsalzen von geeigneten organischen Carbonsäuren, z.B. dem Natriumsalz der $\alpha$-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von Isomeren können nach an sich bekannten Methoden in die einzelnen Isomeren aufgetrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren.

Die Spaltung von erhaltenen Racematen in ihre optischen Antipoden kann auf verschiedenen Wegen erfolgen.

Einer dieser Wege besteht darin, dass man ein Racemat mit einem optisch aktiven Hilfsstoff reagieren lässt, das dabei entstandene Gemisch zweier diastereomerer Verbindungen mit Hilfe von geeigneten physikalisch-chemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I, worin $R_3$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-)-2-Octanol verestert sein oder werden, worauf nach erfolgter Isolierung des gewünschten Diastereomeren die Carboxylgruppe freigesetzt wird.

Zur Racemattrennung kann auch die Hydroxygruppe mit optisch aktiven Säuren oder deren reaktionsfähigen, funktionellen Derivaten verestert

werden, wobei sich diastereomere Ester bilden. Solche Säuren sind beispielsweise (-)-Abietinsäure, D(+)- und L(-)-Aepfelsäure, N-acylierte optisch aktive Aminosäure, (+)- und (-)-Camphansäure, (+)- und (-)-Ketopinsäure, L(+)-Ascorbinsäure, (+)-Camphersäure, (+)-Campher-10-sulfonsäure(β), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-benzoyl- und Di-O-p-toluylderivate.

Durch Umsetzung mit optisch aktiven Isocyanaten, wie mit (+)- oder (-)-1-Phenyläthylisocyanat, können Verbindungen der Formel (I), worin $R_3$ geschütztes Carboxy und $R_2$ Hydroxy bedeutet, in ein Gemisch diastereomerer Urethane umgewandelt werden.

Basische Racemate, z.B. Verbindungen der Formel I, worin der Rest $R_4$ durch Amino substituiert ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden gegenüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produktes aus.

Die Auftrennung von Diastereomeren in die einzelnen Racemate und der Racemate in die optischen Antipoden, kann auf einer beliebigen Verfahrensstufe, d.h. z.B. auch auf der Stufe der Ausgangsverbindungen der Formeln II oder III oder auf einer beliebigen Stufe des nachstehend beschriebenen Verfahrens zur Herstellung der Ausgangsverbindungen der Formel II, durchgeführt werden.

Bei allen nachträglichen Umwandlungen erhaltener Verbindungen der Formel I werden solche Reaktionen bevorzugt, die unter neutralen, alkalischen oder schwach sauren Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine, wie nachstehend beschrieben, gegebenenfalls substituierte Methylidengruppe ist, durch Ozonisierung und anschliessende Reduktion des gebildeten Ozonids, analog dem weiter unten angegebenen Verfahren (Stufe 3.3), in situ hergestellt werden, worauf in der Reaktionslösung die Cyclisierung zur Verbindung der Formel I erfolgt.

Die Ausgangsverbindungen der Formeln II und IV und die Vorstufen können, wie in dem Reaktionsschemata I, II und III angegeben, hergestellt werden:

Reaktionsschema I

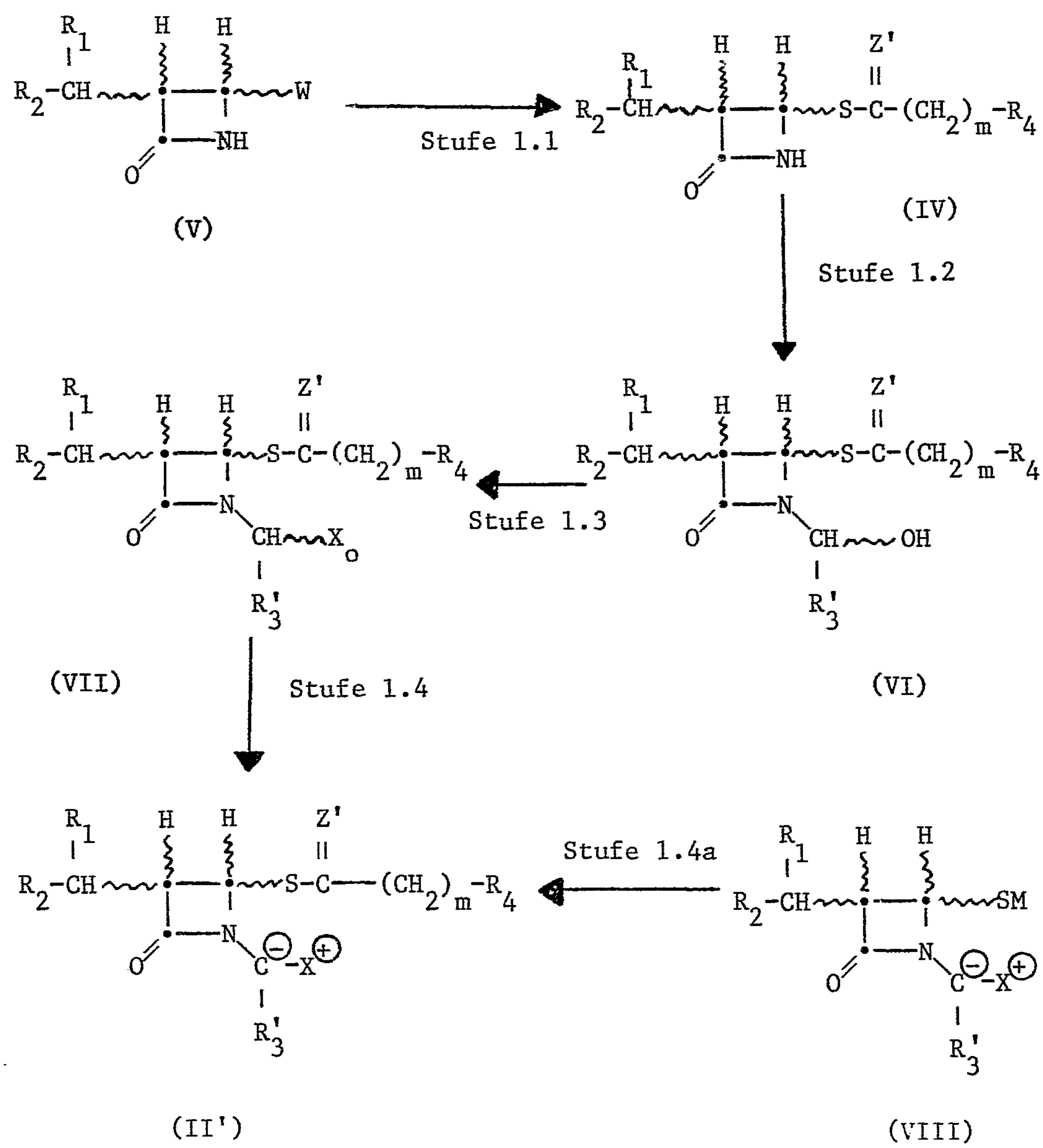


(V) (IV) (VII) (VI) (II') (VIII)

In den Verbindungen der Formeln IV, VI, VII und II' ist Z' Sauerstoff, Schwefel oder auch eine gegebenenfalls durch einen oder zwei Substituenten Y substituierte Methylidengruppe, die durch Oxidation in eine Oxogruppe Z überführt werden kann. Ein Substituent Y dieser Methylidengruppe ist ein organischer Rest, beispielsweise gegebenenfalls substituiertes Niederalkyl, z.B. Methyl oder Aethyl, Cycloalkyl, z.B. Cyclopentyl oder Cyclohexyl, Phenyl oder Phenylniederalkyl, z.B. Benzyl, oder insbesondere eine, inklusive mit einem optisch aktiven Alkohol, wie 1-Menthol, veresterte Carboxylgruppe, z.B. einer der unter $R_3$ erwähnten gegebenenfalls substituierten Niederalkoxycarbonyl- oder Arylmethoxycarbonylreste oder auch 1-Menthyloxycarbonyl. Die Methylidengruppe Z' trägt bevorzugt einen der genannten Substituenten. Hervorzuheben sind die Methoxycarbonylmethyliden-, Aethoxycarbonylmethyliden- und die 1-Menthyloxycarbonylmethylidengruppe Z'. Letztere kann zur Herstellung optisch aktiver Verbindungen der Formeln IV, VI, VII und II verwendet werden.

In den Verbindungen der Formeln IV bis VIII sowie II' ist der Rest $R_2$ bevorzugt eine der genannten geschützten Hydroxygruppen, z.B. gegebenenfalls substituiertes 1-Phenylniederalkoxy, gegebenenfalls substituiertes Phenylniederalkoxycarbonyloxy, oder trisubstituiertes Silyloxy.

<u>Stufe 1.1</u>: Ein Thio-azetidinon der Formel IV wird erhalten, indem man ein 4-W-Azetidinon der Formel V, worin W eine nucleofuge Abgangsgruppe bedeutet, mit einer Mercaptoverbindung der Formel

$$R_4-(CH_2)_m-C(=Z')-SH$$

oder einem Salz, z.B. einem Alkalimetall-, wie Natrium- oder Kaliumsalz davon, behandelt, und, wenn gewünscht, in einer erhältlichen Verbindung der Formel IV, worin $R_2$ Hydroxy ist, Hydroxy in geschütztes Hydroxy überführt.

Die nucleofuge Abgangsgruppe W in einem Ausgangsmaterial der Formel V ist ein durch den nucleophilen Rest

$$R_4-(CH_2)_m-C(=Z')-S-$$

ersetzbarer Rest. Solche Gruppen W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure, inklusive einer optisch aktiven Carbonsäure, und bedeutet beispielsweise Niederalkanoyl, z.B. Acetyl oder Propionyl, gegebenenfalls substituiertes Benzoyl, z.B. Benzoyl oder 2,4-Dinitrobenzoyl, Phenylniederalkanoyl, z.B. Phenylacetyl, oder den Acylrest einer der oben genannten optisch aktiven Säuren. In einem Sulfonylrest $R_o-SO_2-$ ist $R_o$ beispielsweise gegebenenfalls durch Hydroxy substituiertes Niederalkyl, wie Methyl, Aethyl oder 2-Hydroxyäthyl, ferner auch entsprechendes substituiertes optisch aktives Niederalkyl, z.B. (2R)- oder (2S)-1-Hydroxyprop-2-yl, durch einen optisch aktiven Rest substituiertes Methyl, wie Campheryl, oder Benzyl, oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Die nucleophile Substitution kann unter neutralen oder schwach basischen Bedingungen in Gegenwart von Wasser und gegebenenfalls einem mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, Acetonitril und ähnliche verwendet werden. Die Reaktion wird üblicherweise bei Raumtemperatur

durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl optisch inaktive cis- oder trans-Verbindungen der Formel V, als auch Mischungen davon, oder entsprechende optisch aktive Verbindungen eingesetzt werden. Die eintretende Gruppe $R_4-(CH_2)_m-C(=Z')-S-$ wird von der Gruppe $(R_1,R_2)CH-$ bevorzugt in die trans-Stellung dirigiert, unabhängig davon, ob W zur Gruppe $(R_1,R_2)CH-$ in cis- oder trans-Stellung steht. Obwohl die trans-Isomeren überwiegend gebildet werden, können doch gelegentlich auch die cis-Isomeren isoliert werden. Die Auftrennung der cis- und trans-Isomeren erfolgt wie oben beschrieben, nach konventionellen Methoden, insbesondere durch Chromatographie und/oder durch Kristallisation.

Die nachträgliche Ozonisierung einer Methyliden-Gruppe Z' kann wie weiter unten angegeben durchgeführt werden. Ein erhaltenes Racemat der Formel IV kann in die optisch aktiven Verbindungen getrennt werden.

Ein Azetidinon der Formel V, worin $R_2$ und W jeweils Acetoxy und $R_1$ Wasserstoff bedeuten, ist in der Deutschen Offenlegungsschrift Nr. 29 50 898 beschrieben.
Andere Azetidinone der Formel V können nach an sich bekannten Verfahren hergestellt werden, beispielsweise indem man einen Vinylester der Formel $(R_1,R_2)CH-CH=CH-W$ mit Chlorsulfonylisocyanat umsetzt und das entstandene Cycloaddukt mit einem Reduktionsmittel, z.B. Natriumsulfit, umsetzt. Bei dieser Synthese werden gewöhnlich Mischungen von cis- und trans-Isomeren erhalten, die gewünschtenfalls in die reinen cis- oder trans-Isomeren, z.B. durch Chromatographie und/oder Kristallisation oder Destillation, aufgetrennt werden können. Die reinen cis- und trans-Isomeren liegen als Racemate vor und können in ihre optischen

Antipoden getrennt werden, beispielsweise wenn Acyl in einem Acyloxyrest W in Verbindungen der Formel V von einer optisch aktiven Säure stammt. Die Verbindungen der Formel V, insbesondere ihre optisch aktiven Vertreter, können auch nach den unten in den Reaktionsschemata II und III angegebenen Verfahren hergestellt werden.

Stufe 1.2: Eine α-Hydroxycarbonsäureverbindung der Formel VI wird erhalten, indem man eine Verbindung der Formel IV mit einer Glyoxylsäure-Verbindung der Formel OHC-$R_3'$ oder einem geeigneten Derivat davon, wie einem Hydrat, Hemihydrat oder Halbacetal, z.B. einem Halbacetal mit einem Niederalkanol, z.B. Methanol oder Aethanol, umsetzt und, wenn gewünscht, in einer erhältlichen Verbindung der Formel VI, worin $R_2$ Hydroxy ist, Hydroxy in geschütztes Hydroxy überführt.

Die Verbindung VI erhält man üblicherweise als Gemisch der beiden Isomeren (bezüglich der Gruppierung >CH~OH). Man kann aber auch die reinen Isomeren davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100°C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydrationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

In die Reaktion können sowohl reine optisch inaktive cis- oder trans-Verbindungen der Formel IV, als auch Mischungen davon, oder entsprechende optisch aktive Verbindungen eingesetzt werden. Ein erhaltenes Racemat der Formel VI kann in die optisch aktiven Verbindungen getrennt werden.

Stufe 1.3: Verbindungen der Formel VII, worin $X_o$ für eine reaktionsfähige veresterte Hydroxygruppe, insbesondere für Halogen oder organisches Sulfonyloxy steht, werden hergestellt, indem man in einer Verbindung der Formel VI die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkansulfonyloxy, z.B. Methansulfonyloxy, oder Arensulfonyloxy, z.B. Benzol- oder 4-Methylbenzolsulfonyloxy, umwandelt.

In den Ausgangsverbindungen der Formel VI steht $R_2$ vorzugsweise für eine geschützte Hydroxygruppe.

Die Verbindungen der Formel VII kann man in Form von Gemischen der Isomeren (bezüglich der Gruppierung $>CH\sim X_o$) oder in Form von reinen Isomeren erhalten.

Die obige Reaktion wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphondibromid oder -dijodid, oder einem geeigneten organischen Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder "Polystyrol-Hünigbase", oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig, unter Kühlen und/oder in der Atmosphäre eines Inertgases, wie Stickstoff.

In einer so erhältlichen Verbindung der Formel VII kann eine reaktionsfähige veresterte Hydroxygruppe $X_o$ in an sich bekannter Weise in eine andere reaktionsfähige veresterte Hydroxygruppe umgewandelt werden. So kann man z.B. ein Chloratom durch Behandeln der entsprechenden Chlorverbindung mit einem geeigneten Bromid- oder Jodidsalz, wie Lithiumbromid oder -jodid, vorzugsweise in Gegenwart eines geeigneten Lösungsmittels, wie Aether, durch ein Brom- bzw. Jodatom austauschen.

In die Reaktion können sowohl reine optische inaktive cis- oder trans-Verbindungen der Formel VI als auch Mischungen davon, oder entsprechende optisch aktive Verbindungen eingesetzt werden. Ein erhaltenes Racemat der Formel VII kann in die optisch aktiven Verbindungen getrennt werden.

Stufe 1.4: Das Ausgangsmaterial der Formel II' wird erhalten, indem man eine Verbindung der Formel VII, worin $X_o$ für eine reaktionsfähige veresterte Hydroxygruppe steht, mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, wobei man je nach Wahl des Reagenzes eine Verbindung der Formel II (bzw. II') oder IIa erhalten kann.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Aethers, z.B. Dioxan, Tetrahydrofuran oder Diäthylenglykol-dimethyläther, oder

eines Lösungsmittelgemisches vorgenommen. Je nach Reaktionsfähigkeit arbeitet man unter Kühlen oder bei erhöhter Temperatur, etwa zwischen -10° und +100°, bevorzugt bei etwa 20° bis 80°, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins, wie Triäthylamin, Diisopropyl-äthyl-amin oder "Polystyrol-Hünigbase", und gelangt so direkt zum Ylid-Ausgangsmaterial der Formel II (bzw. II'), das aus dem entsprechenden Phosphoniumsalz gebildet wird.

In die Reaktion können sowohl reine, optisch inaktive cis- oder trans-Verbindungen der Formel VII, als auch Mischungen davon, oder entsprechende optisch aktive Verbindungen eingesetzt werden. Ein erhaltenes Racemat der Formel II kann in die optisch aktiven Verbindungen getrennt werden.

Stufe 1.4a

Eine Ausgangsverbindung der Formel II',worin Z' für Oxo steht, kann weiterhin erhalten werden, indem man ein Mercaptid der Formel VIII, worin M für ein Metallkation steht, mit einem den Rest $R_4-(CH_2)_m-C(=O)-$ einführenden Acylierungsmittel behandelt.

In dem Ausgangsmaterial der Formel VIII ist das Metallkation M beispielsweise ein Kation der Formel $M^+$ oder $M^{2+}/2$, wobei $M^+$ insbesondere für ein Silberkation und $M^{2+}$ z.B. für das zweiwertige Kation eines geeigneten Uebergangsmetalls, z.B. Kupfer, Blei oder Quecksilber, steht.

Ein den Rest $R_4-(CH_2)_m-C(=O)-$ einführendes Acylierungsmittel ist z.B. die Säure $R_4-(CH_2)_m-COOH$ oder ein reaktionsfähiges funktionelles Derivat davon, wie ein Säurehalogenid, z.B. Chlorid oder Bromid, Azid oder Anhydrid davon.

Die Acylierung erfolgt, wenn die freie Säure der Formel $R_4-(CH_2)_m-COOH$ eingesetzt wird, z.B. in Gegenwart eines geeigneten wasserentziehenden Mittels, wie eines Carbodiimids, z.B. N,N'-Dicyclohexylcarbodiimid, oder, wenn ein Säurederivat eingesetzt wird, in Gegenwart eines geeigneten säurebindenden Mittels, wie einer tertiären aliphatischen oder aromatischen Base, z.B. Triäthylamin, Pyridin oder Chinolin, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel VIII können beispielsweise hergestellt werden, indem man ein Azetidinon der Formel

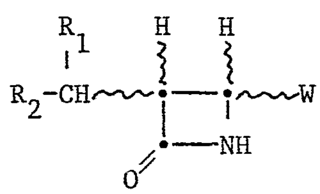

(V) ,

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

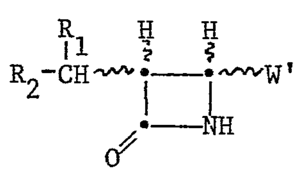

(V')

überführt, worin W' Triphenylmethylthio oder Niederalkanoylthio, z.B. Acetylthio, bedeutet, diese analog dem in den Reaktionsstufen 1.2, 1.3 und 1.4 beschriebenen Verfahren in eine Verbindung der Formel

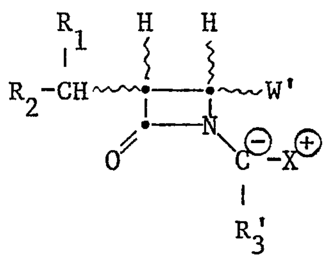

(VIII')

überführt und diese in Gegenwart einer Base, z.B. Pyridin oder Tri-n-butylamin, in einem geeigneten Lösungsmittel z.B. Diäthyläther oder Methanol, mit einem Salz der Formel MA, worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Die Ylide der Formel II', worin Z' Sauerstoff oder Schwefel ist, können direkt in die Cyclisierungsreaktion zur Herstellung der Endprodukte der Formel I eingesetzt werden. Man kann aber auch in Verbindungen der Formel II', worin $R_2$ eine geschützte Hydroxygruppe, z.B. eine hydrolytisch leicht spaltbare geschützte Hydroxygruppe, wie trisubstituiertes Silyloxy, ist, zuerst die Hydroxyschutzgruppe abspalten und dann die erhaltene Verbindung der Formel II', worin $R_2$ Hydroxy ist, in die Cyclisierungsreaktion einsetzen.

In den Verbindungen II', IV, VI und VII kann eine gegebenenfalls substituierte Methylidengruppe Z' durch Ozonisierung und anschliessender Reduktion des gebildeten Ozonids, gemäss dem nachfolgend in der Stufe 3.3 beschriebenen Verfahren, in die Oxogruppe Z überführt werden.

Ausgangsverbindungen der Formel V, worin W ein Sulfonylrest $HO-A-SO_2-$ ist, können auch nach dem folgenden Reaktionsschema II hergestellt werden.

Reaktionsschema II

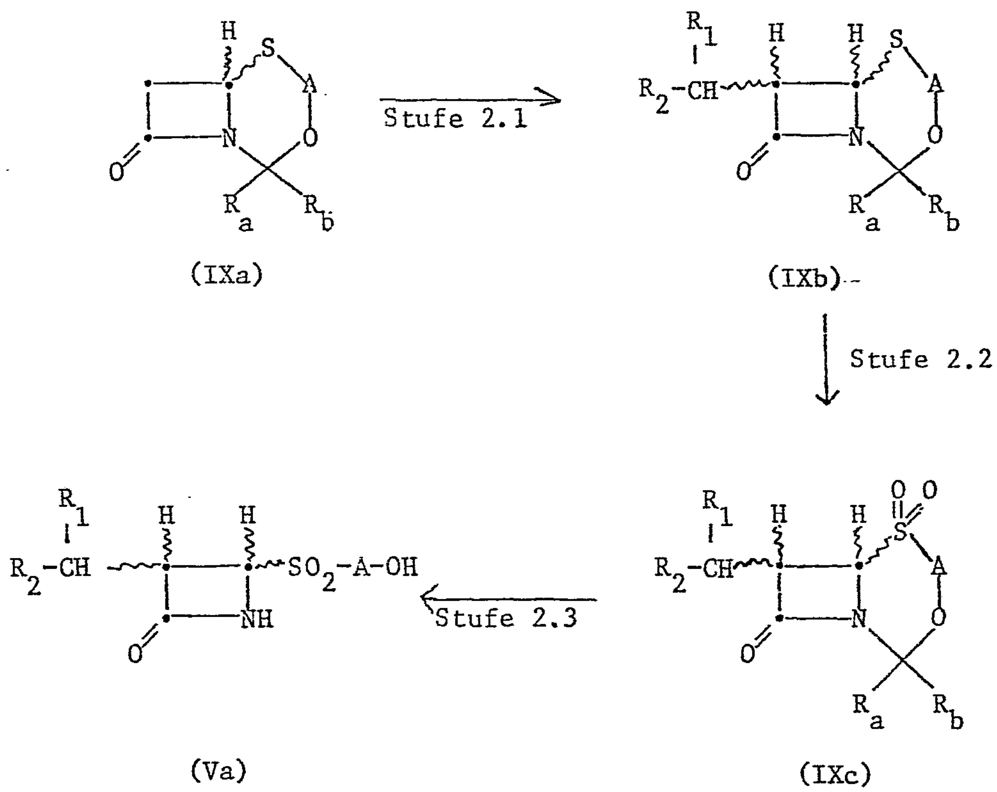


(IXa) (IXb) (IXc) (Va)

In den Verbindungen der Formeln (IXa)-(IXc) und (Va) stellt A einen Niederalkylenrest mit 2 bis 3 Kohlenstoffatomen zwischen den beiden Heteroatomen dar und ist in erster Linie Aethylen oder 1,2-Propylen, kann jedoch auch 1,3-Propylen, 1,2-, 2,3- oder 1,3-Butylen sein.

In den Verbindungen der Formeln (IXa)-(IXc) steht jeder der Reste $R_a$ und $R_b$ für Wasserstoff oder für einen über ein Kohlenstoffatom mit dem Ringkohlenstoffatom verbundenen organischen Rest, wobei die beiden Reste $R_a$ und $R_b$ untereinander verknüpft sein können, in erster Linie für Wasserstoff, Niederalkyl, z.B. Methyl, Aethyl, n-Propyl oder Isopropyl, gegebenenfalls substituiertes Phenyl, oder Phenylniederalkyl, z.B. Benzyl, oder, wenn zusammengenommen, Niederalkylen vorzugsweise mit 4 bis 6 Kohlenstoffatomen, z.B. 1,4-Butylen oder 1,5-Pentylen.

In den Verbindungen der Formel (IXb), (IXc) und (Va) ist der Rest $R_2$ Hydroxy oder vorzugsweise eine der genannten geschützten Hydroxygruppen, z.B. gegebenenfalls substituiertes 1-Phenylniederalkoxy, gegebenenfalls substituiertes Phenylniederalkoxycarbonyloxy oder trisubstituiertes Silyloxy.

Stufe 2.1

Eine Verbindung der Formel (IXb) wird erhalten, indem man eine bicyclische Verbindung der Formel (IXa) mit einem Metallierungreagenz und einem den Rest $(R_1,R_2)CH-$ einführenden Elektrophil umsetzt und anschliessend das entstandene Produkt mit einer Protonenquelle behandelt.

Geeignete Metallierungsreagenzien sind z.B. substituierte und unsubstituierte Alkalimetallamide, Alkalimetallhydride oder Alkalimetallniederalkylverbindungen, worin das Alkalimetall z.B. Natrium oder insbesondere Lithium ist, z.B. Natrium- oder Lithiumamid, Lithium-bistrimethylsilylamid, Natriumhydrid, Lithiumhydrid und bevorzugt Lithiumdiisopropylamid und Butyllithium.

Den Rest $(R_1,R_2)CH-$ einführende Elektrophile sind beispielsweise Verbindungen der Formel $R_1-CH=O$ oder funktionelle Derivate davon der Formel $(R_1,R_2)CH-X$, worin X eine nucleofuge Abgangsgruppe, insbesondere Halogen, z.B. Chlor, Brom oder Jod, oder Sulfonyloxy, z.B. Methansulfonyloxy oder 4-Toluolsulfonyloxy, darstellt. Bevorzugte den Rest $(R_1,R_2)CH-$ einführende Elektrophile sind Formaldehyd, gegebenenfalls substituiertes Benzyloxymethylchlorid und Acetaldehyd.

Für die Metallierungsreaktion geeignete Lösungsmittel dürfen keinen aktiven Wasserstoff enthalten und sind z.B. Kohlenwasserstoffe, z.B. Hexan, Benzol, Toluol oder Xylol, Aether, z.B. Diäthyläther, Tetrahydrofuran oder Dioxan, oder Säureamide, z.B. Hexamethylphosphorsäuretriamid.

Das metallierte Zwischenprodukt braucht nicht isoliert, sondern kann im Anschluss an die Metallierungsreaktion mit dem den Rest $(R_1,R_2)CH$- einführenden Elektrophil umgesetzt werden. Die Metallierungsreaktion erfolgt bei Temperaturen von etwa -100°C bis etwa Raumtemperatur, vorzugsweise unterhalb -30°C, vorzugsweise in einer Inertgas-, wie Stickstoffatmosphäre. Die weitere Umsetzung kann unter den gleichen Bedingungen erfolgen. Dabei wird Formaldehyd vorzugsweise in gasförmiger, monomerer Form in das Reaktionsgemisch eingeleitet. Monomerer Formaldehyd ist beispielsweise durch thermische Depolymerisation von Paraformaldehyd oder durch thermische Zersetzung von Formaldehydcyclohexylhemiacetal erhältlich.

Für die Metallierungsreaktion können sowohl die Antipoden von Verbindungen der Formel (IXa) als auch deren racemische oder diastereomere Mischungen eingesetzt werden.

Der Angriff des den Rest $(R_1,R_2)CH$- einführenden Elektrophils an das Substrat erfolgt im allgemeinen stereospezifisch. Wird als Ausgangsmaterial ein Azetidinon der Formel (IXa) mit R-Konfiguration am Kohlenstoffatom 4 des Azetidinon-Rings benutzt, so entsteht überwiegend eine Verbindung der Formel (IXb) mit R-Konfiguration am Kohlenstoffatom 4 und S-Konfiguration am Kohlenstoffatom 3 des Azetidinon-Rings, d.h., der Angriff des Elektrophils erfolgt überwiegend trans-ständig.

Nach der Umsetzung wird das Reaktionsprodukt mit einer Protonenquelle behandelt, z.B. mit Wasser, einem Alkohol, wie Methanol oder Aethanol, einer organischen oder anorganischen Säure, z.B. Essigsäure, Salzsäure, Schwefelsäure, oder einer ähnlichen Protonen-abgebenden Verbindung, bevorzugt wiederum bei niederen Temperaturen.

In einer erhaltenen Verbindung der Formel (IXb), worin $R_2$ Wasserstoff ist, kann man die Hydroxygruppe in an sich bekannter Weise, z.B. durch Veräthern oder Verestern, insbesondere wie oben beschrieben, schützen.

Die Herstellung von optisch aktiven und optisch inaktiven Ausgangsverbindungen der Formel (IXa) ist beispielsweise in der europäischen Patentanmeldung Nr. 23887 beschrieben.

Stufe 2.2

Ein Sulfon der Formel (IXc) kann hergestellt werden, indem man die Thioverbindung der Formel (IXb) mit einem Oxidationsmittel behandelt und gewünschtenfalls eine verfahrensgemäss erhältliche Verbindung der Formel (IXc), worin $R_2$ Hydroxy ist, in eine Verbindung der Formel (IXc) überführt, worin $R_2$ eine geschützte Hydroxygruppe ist.

Geeignete Oxidationsmittel sind beispielsweise Wasserstoffperoxid, organische Persäuren, insbesondere aliphatische oder aromatische Percarbonsäuren, z.B. Peressigsäure, Perbenzoesäure, Chlorperbenzoesäure, z.B. 3-Chlorperbenzoesäure, oder Monoperphthalsäure, oxidierende anorganische Säuren und deren Salze, z.B. Salpetersäure, Chromsäure, Kaliumpermanganat, oder Alkalimetallhypochlorite, z.B. Natriumhypochlorit. Die Ueberführung kann aber auch durch anodische Oxidation erfolgen.

Die Oxidation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, z.B. Diäthyläther Dioxan oder Tetrahydrofuran, einem Amid, z.B. Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur, oder unter Kühlen oder leichtem Erwärmen, d.h. bei etwa -20°C bis etwa +90°C, bevorzugt bei etwa Raumtemperatur durchgeführt. Die Oxidation kann auch stufenweise durchgeführt werden, indem zunächst bei niederer Temperatur, d.h. bei etwa -20°C bis etwa 0°C, bis zum Sulfoxid oxidiert wird, das gegebenenfalls isoliert wird, worauf in einem zweiten Schritt, bevorzugt bei höherer Temperatur, etwa bei Raumtemperatur, das Sulfoxid zum Sulfon der Formel (IXc) oxidiert wird.

Zur Aufarbeitung kann gegebenenfalls noch vorhandenes überschüssiges Oxidationsmittel durch Reduktion, insbesondere durch Behandeln mit einem Reduktionsmittel, wie einem Thiosulfat, z.B. Natriumthiosulfat, zerstört werden.

In die Reaktion können sowohl optisch inaktive Verbindungen der Formel (IXb) als auch entsprechende optisch aktive Verbindungen, insbesondere solche mit 3S,4R-Konfiguration im Azetidinon-Ring, eingesetzt werden.

Stufe 2.3

Verbindungen der Formel (Va) können hergestellt werden, indem man ein bicyclisches Amid der Formel (IXc) mit einem geeigneten Solvolysereagenz solvolysiert und, wenn gewünscht, in einer verfahrensgemäss erhältlichen Verbindung der Formel (Va) eine freie Hydroxygruppe $R_2$ in eine geschützte Hydroxygruppe $R_2$ umwandelt.

Geeignete Solvolysereagenzien sind beispielsweise organische Säuren, z.B. Niederalkancarbonsäuren, wie Ameisensäure oder Essigsäure, oder Sulfonsäuren, z.B. 4-Toluolsulfonsäure oder Methansulfonsäure, Mineralsäuren, z.B. Schwefel- oder Salzsäure, ferner Niederalkanole, z.B. Methanol oder Aethanol, oder Niederalkandiole, z.B. Aethylenglykol.

Die genannten Solvolysereagenzien werden unverdünnt oder mit Wasser verdünnt zugegeben. Die Solvolyse kann auch mit reinem Wasser durchgeführt werden. Die Solvolyse mit dem sauren Reagenz findet bevorzugt in wässriger Lösung dieses Reagenzes und bei Temperaturen von etwa -20°C bis etwa 150°C, bevorzugt bei Zimmertemperatur bis 110°C statt.

In die Reaktion können sowohl optisch inaktive Verbindungen der Formel (IXc), z.B. Racemate oder Diastereomerengemische, als auch entsprechende optisch aktive Verbindungen, insbesondere solche mit 3S,4R-Konfiguration im Azetidinon-Ring, eingesetzt werden.

Erhaltene Isomerengemische von Verbindungen der Formel (IXb), (IXc) und (Va), wie Racemate oder Diastereomerengemische, können in an sich bekannter Weise, z.B. wie oben beschrieben, in die einzelnen Isomeren, wie Antipoden, getrennt werden.

Erfindungsgemäss verwendbare optisch aktive trans-Verbindungen der Formel (V) können auch nach dem folgenden Reaktionsschema III hergestellt werden:

Reaktionsschema III

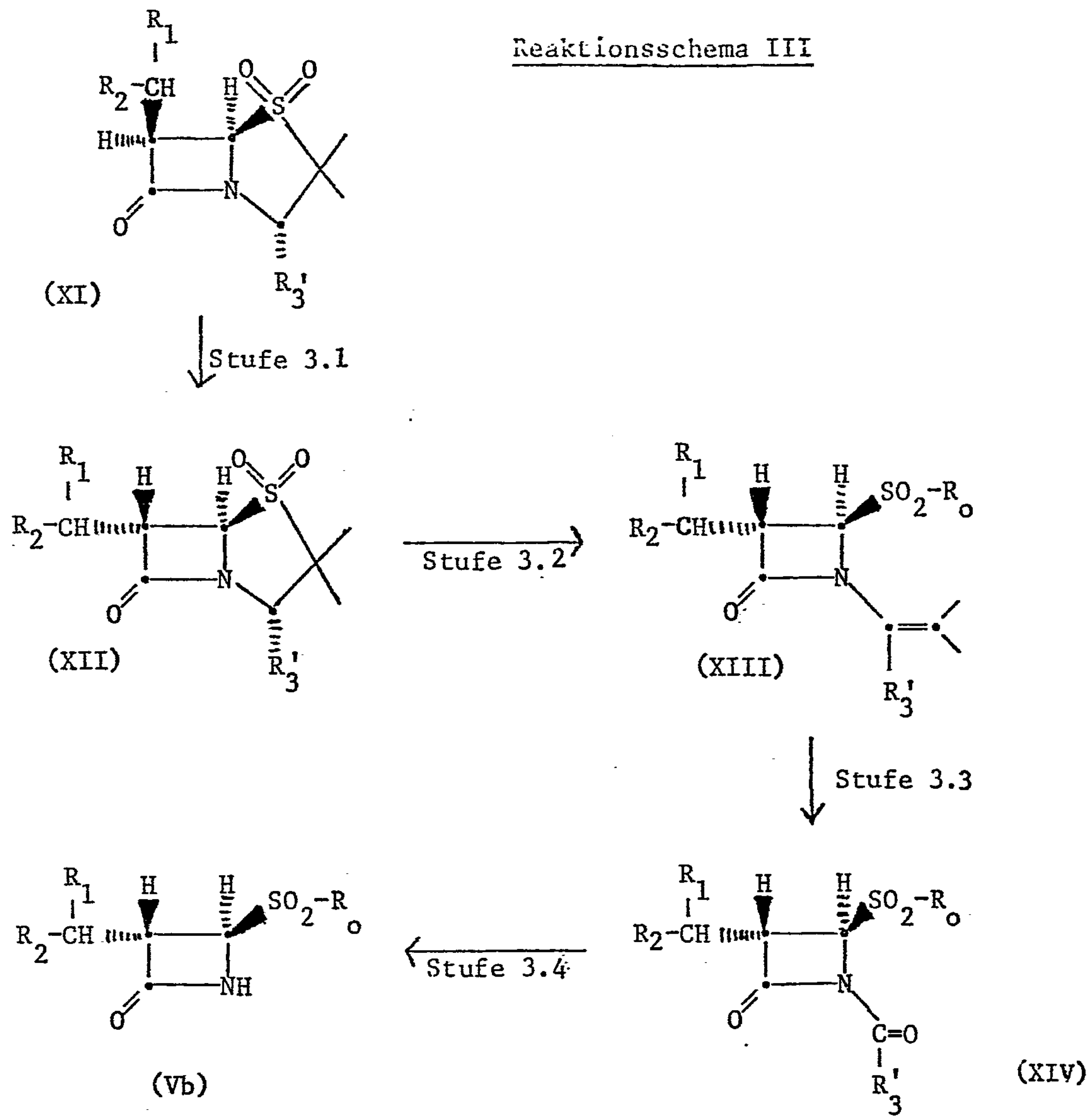


In den Verbindungen der Formel (XI) - (XIV) und (Vb) steht $R_2$ für Hydroxy oder insbesondere für eine geschützte Hydroxygruppe.

Stufe 3.1

Verbindungen der Formel (XII) sind bekannt oder können auf an sich bekannte Weise hergestellt werden. Sie können auch nach einem neuen Verfahren hergestellt werden, indem man eine Verbindung der Formel (XI) epimerisiert und, wenn gewünscht, in einer verfahrensgemäss erhältlichen Verbindung der Formel (XII) eine geschützte Hydroxygruppe $R_2$ in eine andere geschützte Hydroxygruppe $R_2$ überführt.

Die Epimerisierung erfolgt beispielsweise in Gegenwart eines basischen Mittels, wie eines Amins, z.B. eines Triniederalkylamins, z.B. Triäthylamin oder Aethyl-diisopropylamin, eines tertiären Amins, z.B. N,N-Dimethylanilin, eines aromatischen Amins, z.B. Pyridin, oder eines bicyclischen Amins, z.B. 1,5-Diazabicyclo[5,4,0]undec-5-en oder 1,5-Diazabicyclo[4,3,0]non-5-en, oder eines Alkalimetallniederalkanolats, z.B. Natriummethanolat, Natriumäthanolat oder Kalium-tert.-butanolat, in einem inerten Lösungsmittel, beispielsweise einem Aether, z.B. Diäthyläther, Dimethoxyäthan, Tetrahydrofuran oder Dioxan, Acetonitril oder Dimethylformamid, gegebenenfalls bei etwas erhöhter oder erniedrigter Temperatur, z.B. bei 0°C bis 50°C, vorzugsweise bei Raumtemperatur.

In den verfahrensgemäss erhältlichen Verbindungen der Formel (XII) kann eine geschützte Hydroxygruppe $R_2$ durch eine andere geschützte Hydroxygruppe $R_2$ beispielsweise eine hydrogenolytisch spaltbare geschützte Hydroxygruppe durch eine solvolytisch spaltbare geschützte Hydroxygruppe, ersetzt werden. Hydroxyschutzgruppen sind insbesondere die oben genannten, hydrogenolytisch abspaltbare Schutzgruppen beispielsweise wie angegeben substituiertes 1-Phenylniederalkyl oder Phenylniederalkoxycarbonyl, solvolytisch abspaltbare Schutzgruppen beispielsweise wie angegeben trisubstituiertes Silyl.

Die Umsetzung kann so durchgeführt werden, dass man zunächst die hydrogenolytisch abspaltbare Hydroxyschutzgruppe entfernt und in die entstandene Verbindung der Formel XII, worin $R_2$ Hydroxy ist, eine solvolytisch abspaltbare Hydroxyschutzgruppe einführt.

Die Abspaltung der hydrogenolytisch abspaltbaren Schutzgruppe erfolgt z.B. mit Wasserstoff oder einem Wasserstoffdonator, z.B. Cyclohexen oder Cyclohexadien, in Gegenwart eines

Hydrierungskatalysators, wie eines Palladiumkatalysators, z.B. Palladium auf Kohle, in einem inerten Lösungsmittel, wie einem halogenierten Kohlenwasserstoff, z.B. Methylenchlorid, einem Niederalkanol, z.B. Methanol oder Aethanol, einem Aether, z.B. Dioxan oder Tetrahydrofuran, oder auch in Wasser oder in Gemischen davon, bei einer Temperatur von etwa 0° bis etwa 80°C, vorzugsweise bei Raumtemperatur.
Die Abspaltung kann auch mit einem reduzierenden Metall, wie Zink, oder einer reduzierenden Metallegierung, z.B. Kupfer-Zink-Legierung, in Gegenwart eines Protonen-abgebenden Mittels, wie einer organischen Säure, z.B. Essigsäure, oder auch eines Niederalkanols, z.B. Aethanol, durchgeführt werden.

Die Einführung der solvolytisch abspaltbaren Hydroxyschutzgruppe erfolgt beispielsweise mit einer Verbindung der Formel $R_2'-X_3$, worin $R_2'$ die Hydroxyschutzgruppe und $X_3$ z.B. eine reaktionsfähige veresterte Hydroxygruppe, beispielsweise Halogen, z.B. Chlor, Brom oder Jod, oder Sulfonyloxy, wie Methansulfonyloxy, Benzolsulfonyloxy oder 4-Toluolsulfonyloxy, bedeutet.

Die Umsetzung erfolgt in einem inerten Lösungsmittel, wie einem Aether, z.B. Diäthyläther, Dioxan oder Tetrahydrofuran, einem Kohlenwasserstoff, z.B. Benzol oder Toluol, einem Halogenkohlenwasserstoff, z.B. Methylenchlorid, in Dimethylsulfoxid oder Acetonitril, in Gegenwart eines basischen Kondensationsmittels, wie eines Alkalimetallhydroxids oder -carbonats, z.B. Natrium- oder Kaliumhydroxid oder Natrium- oder Kaliumcarbonat, eines Alkalimetallamids oder -hydrids, z.B. Natriumamid oder Natriumhydrid, eines Alkalimetallniederalkanolats, z.B. Natriummethanolat oder -ethanolat oder Kalium-tert.-butanolat, oder eines Amins, z.B. Triäthylamin, Pyridin oder Imidazol, bei Raumtemperatur oder erhöhter oder erniedrigter Temperatur, z.B. bei etwa -20°C bis etwa 80°C, bevorzugt bei Raumtemperatur.

Ausgangsverbindungen der Formel (XI) sind beispielsweise aus der Deutschen Offenlegungsschrift 3 039 504 und aus der britischen Patentanmeldung 20 61 930 bekannt.

Stufe 3.2

Eine Verbindung der Formel (XIII) kann hergestellt werden, indem man eine Penam-Verbindung der Formel (XII) mit einem basischen Mittel und mit einem den Rest $R_o$ einführenden Veresterungsmittel behandelt.

Ein geeignetes basisches Mittel ist beispielsweise eines der unter Stufe 3.1 genannten basischen Mittel, insbesondere eines der genannten bicyclischen Amine, ferner auch ein Alkalimetallamid oder -hydrid, z.B. Natriumamid oder Natriumhydrid.

Ein Rest $R_o$ ist beispielsweise einer der unter Stufe 1.1 genannten organischen Reste, insbesondere gegebenenfalls substituiertes Niederalkyl, z.B. Methyl, Aethyl oder 2-Hydroxyäthyl, oder Benzyl.

Ein den Rest $R_o$ einführendes Veresterungsmittel ist z.B. eine Verbindung der Formel $R_o$-$X_4$, worin $X_4$ reaktionsfähiges verestertes Hydroxy, z.B. Halogen, wie Chlor, Brom oder Jod, oder Sulfonyloxy, wie Methansulfonyloxy, Benzolsulfonyloxy oder 4-Toluolsulfonyloxy, bedeutet. Zur Einführung eines 2-Hydroxyäthylrestes ist auch Aethylenoxid geeignet.

Die Umsetzung wird vorzugsweise zweistufig durchgeführt, wobei man in der ersten Stufe die Penam-Verbindung der Formel (XII) mit mindestens äquimolekularen Mengen des basischen Mittels behandelt und ein erhältliches Zwischenprodukt der Formel

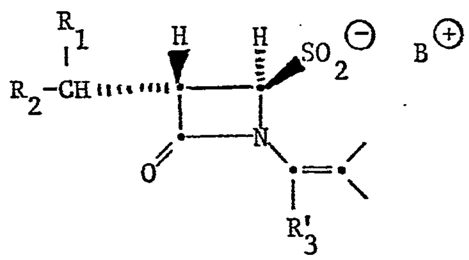

(XIIa) ,

worin $B^{\oplus}$ die protonierte Form (Kation) des basischen Mittels darstellt, vorzugsweise ohne Isolierung aus dem Reaktionsgemisch mit dem Veresterungsmittel umsetzt. Die Reaktion wird in einem inerten Lösungsmittel, beispielsweise einem Aether, z.B. Diäthyläther, Dimethoxyäthan, Tetrahydrofuran oder Dioxan, in Acetonitril, Dimethylformamid oder Hexamethylphosphorsäuretriamid, gegebenenfalls bei etwas erhöhter oder erniedrigter Temperatur, z.B. bei etwa 0°C bis 50°C, vorzugsweise bei Raumtemperatur, durchgeführt. In einer bevorzugten Ausführungsform des Verfahrens wird die Penam-Verbindung der Formel (XII) in situ hergestellt, indem man wie unter Stufe 3.1 beschrieben, eine Verbindung der Formel (XI) zuerst mit katalytischen Mengen des basischen Mittels, z.B. 1,5-Diazabicyclo[5,4,0]undec-5-en, behandelt, und dann mit zumindest äquimolaren Mengen des gleichen basischen Mittels und des Veresterungsmittels weiter zu den Verbindungen der Formel (XIII) umsetzt.

Stufe 3.3

Ein Oxalyl-azetidinon der Formel (XIV) kann hergestellt werden, indem man eine Verbindung der Formel (XIII) ozonisiert und das gebildete Ozonid reduktiv zur Oxo-Verbindung spaltet.

Die Ozonisierung wird üblicherweise mit einem Ozon-Sauerstoff-Gemisch in einem inerten Lösungsmittel, wie einem Niederalkanol, z.B. Methanol oder Aethanol, einem Niederalkanon, z.B. Aceton, einem gegebenenfalls halogenierten Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter

Kühlen, z.B. bei Temperaturen von etwa -80° bis etwa 0°C, durchgeführt.

Ein als Zwischenprodukt erhaltenes Ozonid wird, üblicherweise ohne isoliert zu werden, reduktiv zu einer Verbindung der Formel XIV gespalten, wobei man katalytisch aktivierten Wasserstoff, z.B. Wasserstoff in Gegenwart eines Schwermetallhydrierungskatalysators, wie eines Nickel-, ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit, in Gegenwart eines Wasserstoffdonators, wie einer Säure, z.B. Essigsäure, oder Wasser, oder reduzierende organische Verbindungen, wie Ameisensäure, verwendet. Als Reduktionsmittel können auch Verbindungen zum Einsatz kommen, die leicht in entsprechende Epoxidverbindungen oder Oxide umgewandelt werden können, wobei die Epoxidbildung aufgrund einer C,C-Doppelbindung und die Oxidbindung aufgrund eines vorhandenen Oxid-bildenden Hetero-, wie Schwefel-, Phosphor- oder Stickstoffatoms erfolgen kann. Solche Verbindungen sind z.B. geeignet substituierte Aethenverbindungen (die in der Reaktion in Aethylenoxidverbindungen umgewandelt werden), wie Tetracyanäthylen, oder insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein gegebenenfalls durch Phenyl und/oder Niederalkyl, z.B. Methyl, Aethyl, n-Propyl oder n-Butyl, substituiertes Phosphin (das in der Reaktion in ein Phosphinoxid umgewandelt wird), wie Triniederalkyl-phosphine, z.B. Tri-n-butylphosphin, oder Triphenylphosphin, ferner Triniederalkylphosphite (die in der Reaktion in Phosphorsäuretriniederalkylester

übergeführt werden), üblicherweise in der Form von entsprechenden Alkoholadduktverbindungen, wie Trimethylphosphit, oder Phosphorigsäure-triamide, welche gegebenenfalls Niederalkyl als Substituenten enthalten, wie Hexaniederalkyl-phosphorigsäuretriamide, z.B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z.B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d.h. in Gegenwart eines geeigneten Lösungsmittels oder Lösungsmittelgemisches, sowie unter Kühlen oder leichtem Erwärmen, wobei man vorzugsweise bei Temperaturen von etwa -10°C bis etwa +25°C arbeitet und die Reaktion üblicherweise bei Raumtemperatur abschliesst.

Stufe 3.4

Ein Azetidinon der Formel (Vb) kann hergestellt werden, indem man ein Oxalyl-azetidinon der Formel (XIV) solvolysiert.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z.B. Methanol oder Aethanol, vorzugsweise in Gegenwart von Wasser und eines organischen Lösungsmittels, wie eines Niederalkancarbonsäure-niederalkylesters, z.B. Essigsäureäthylester, vorzugsweise bei Raumtemperatur, wenn notwendig unter Kühlen oder Erwärmen, z.B. bei einer Temperatur von etwa 0° bis etwa 80°C, durchgeführt. Die Hydrazinolyse wird auf konventionelle Weise mit einem substituierten Hydrazin, z.B. mit Phenyl- oder einem Nitrophenylhydrazin, wie 2-Nitrophenylhydrazin, 4-Nitrophenylhydrazin oder 2,4-Dinitrophenylhydrazin, das bevorzugt

in etwa äquimolarer Menge eingesetzt wird, in einem organischen Lösungsmittel, wie einem Aether, z.B. Tetrahydrofuran, Dioxan, Diäthyläther, einem aromatischen Kohlenwasserstoff, wie Benzol oder Toluol, einem halogenierten Kohlenwasserstoff, wie Methylenchlorid, Chlorbenzol oder Dichlorbenzol, einem Ester wie Aethylacetat, und dergleichen, bei Temperaturen zwischen etwa Raumtemperatur und etwa 65°C durchgeführt.

In einer bevorzugten Ausführungsform des Verfahrens geht man von einer Verbindung der Formel (XIII) aus, die wie angegeben ozonisiert und dann reduktiv zum Oxalyl-azetidinon der Formel (XIV) gespalten wird, welches ohne Isolierung aus dem Reaktionsgemisch weiter zum Azetidinon der Formel (Vb) umgesetzt wird.

Bei der Ozonolyse entstehen gegebenenfalls geringe Mengen an Säure, welche die Abspaltung eines solvolytisch leicht abspaltbaren Restes $R_2$, z.B. eines trisubstituierten Silyl-Restes, bewirken können. Die dabei entstehende Verbindung der Formel

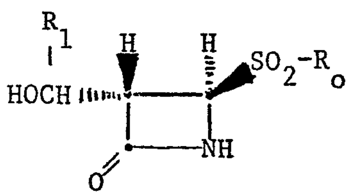

(Vb')

kann, beispielsweise chromatographisch, vom geschützten Azetidinon (Vb) abgetrennt und durch erneute Umsetzung mit dem die Hydroxyschutzgruppe $R_2'$ einführenden Mittel der Formel $R_2'$-$X_3$ in das Azetidinon der Formel (Vb) übergeführt werden.

In den Verbindungen der Formeln (II), (II'), (III), (VI), (VII) und (XII) bis (XIV) kann eine geschützte Carboxylgruppe $R_3'$ nach an sich bekannten Methoden in eine andere geschützte Carboxylgruppe $R_3'$ übergeführt werden, wobei unter Berücksichtigung der gegebenenfalls in diesen Verbindungen enthaltenen weiteren funktionellen Gruppen die

gleichen Methoden zur Anwendung gelangen können, wie zur Umwandlung dieses Substituenten in den Verbindungen der Formel (I) angegeben ist.

Die Erfindung betrifft ebenfalls neue Ausgangsprodukte sowie verfahrensgemäss erhältliche neue Zwischenprodukte, wie diejenigen der Formel (II) bis (VIII), (XIII) und (XIV), sowie die angegebenen Verfahren zu ihrer Herstellung.

Vorzugsweise werden solche Ausgangsstoffe verwendet und die Reaktionsbedingungen so gewählt, dass man zu den vorstehend als besonders bevorzugt aufgeführten Verbindungen gelangt.

Die Verbindungen der Formel I weisen wertvolle pharmakologische Eigenschaften auf, oder können als Zwischenprodukte zur Herstellung von solchen Verbindungen mit wertvollen pharmakologischen Eigenschaften verwendet werden. Verbindungen der Formel I, worin $R_1$ Wasserstoff oder Methyl ist, $R_2$ Hydroxy bedeutet, $R_3$ Carboxyl oder eine unter physiologischen Bedingungen spaltbare veresterte Carboxylgruppe bedeutet und $R_4$ die unter Formel I angegebene Bedeutung hat, und pharmakologisch verwendbare Salze von solchen Verbindungen mit salzbildenden Gruppen haben antibakterielle Wirkungen. Beispielsweise sind sie in vitro gegen grampositive und gramnegative Kokken, z.B. Staphylococcus aureus, Streptococcus pyogenes, Streptococcus pneumoniae, Streptococcus faecalis, Neisseria meningitidis und Neisseria gonorrhoeae, und gegen gramnegative Stäbchenbakterien, wie Enterobacteriaceae, Haemophilus influenzae und Pseudomonas aeruginosa, und Anaerobier, z.B. Bacteroides sp., in minimalen Konzentrationen von ca. 0,01 bis ca. 64 μg/ml wirksam. In vivo, bei der systemischen Infektion der Maus, z.B. durch Staphylococcus aureus oder Streptococcus pyogenes, ergeben sich bei subkutaner oder oraler Applikation $ED_{50}$-Werte von ca. 0,3 bis ca. 30 mg/kg. Beispielsweise weisen Natrium-(5R,6S)-2-[3-(2-methyl-tetrazol-5-yl)-propyl]-6-hydroxymethyl-2-penem-3-carboxylat (Verbindung A) und (5R,6S)-2-[3-(2-Methyl-1,3,4-oxadiazol-5-yl)-propyl]-6-hydroxymethyl-2-penem-3-carbonsäure (Verbindung B) in dem genannten in-vitro-Test

die folgenden Werte auf:

| Organismus | MIC (µg/ml) Verbindung A | Verbindung B |
|---|---|---|
| Staphylococcus aureus 10 B | 0.2 | 0.2 |
| Staphylococcus aureus 2999i+p+ | 0.2 | 0.2 |
| Streptococcus pyogenes Aronson | 0.2 | 0.1 |
| Streptococcus pneumoniae III/84 | 0.05 | 0.05 |
| Streptococcus faecalis D 3 | 32 | 32 |
| Neisseria meningitidis 1316 | 0.01 | 0.05 |
| Neisseria gonorrhoeae 1317-4 | 0.01 | 0.01 |
| Haemophilus influenzae NCTC 4560 | 2 | 2 |
| Escherichia coli 205 | 4 | 1 |
| Escherichia coli 205 R+TEM | 8 | 4 |
| Escherichia coli 16 | 32 | 16 |
| Escherichia coli UB 1005 | 8 | 2 |
| Escherichia coli DC 2 | 2 | 1 |
| Klebsiella pneumoniae 327 | 4 | 2 |
| Serratia marcescens 344 | 64 | 16 |
| Enterobacter cloacae P 99 | 32 | 16 |
| Proteus mirabilis 774 | 4 | 2 |
| Proteus mirabilis 1219 | 8 | 4 |
| Proteus rettgeri 856 | 16 | 4 |
| Proteus morganii 2359 | 2 | 2 |
| Proteus morganii 1518 | 16 | 8 |
| Pseudomonas aeruginosa ATCC 12055 | > 128 | > 32 |
| Pseudomonas aeruginosa K 799/61 | 2 | 2 |
| Clostridium perfringens 194 | 0.2 | 0.1 |
| Bacteroides fragilis L 01 | 0.5 | 0.5 |

In vivo, bei der systemischen Infektion der Maus durch Staphylococcus aureus 10B, ergeben sich z.B. bei subkutaner Applikation $ED_{50}$-Werte von 3-10 mg/kg bei Verbindung A und von 0,3-30 mg/kg bei Verbindung B.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, wobei eine geschützte Carboxylgruppe von einer physiologisch spaltbaren veresterten Carboxylgruppe verschieden ist, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze

davon, wie Magnesium- oder Kalziumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B Mannit, enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen von etwa 0,1 g bis etwa 5 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm,

IR: Infrarotspektrum,

UV: Ultraviolettspektrum,

Smp.: Schmelzpunkt,

DBU: 1,5-Diazabicyclo[5.4.0]undec-5-en,

THF: Tetrahydrofuran,

DMF: Dimethylformamid.

Experimenteller Teil

Beispiel 1: (3S,4R)-3-(tert.Butyl-dimethylsilyloxymethyl)-4-[4-(2—methyl-tetrazol-5-yl)-butyroylthio]-azetidin-2-on

Eine Lösung von 293,5 mg 3-(tert.Butyldimethylsilyloxymethyl)-4-methylsulfonyl-azetidin-2-on in 40 ml abs. THF wird bei Raumtemperatur unter Rühren, tropfenweise mit einem Gemisch aus 242,1 mg 4-(2'-Methyl-tetrazol-5'-yl)-thiobuttersäure, 1,6 ml 1N Natronlauge und 2,3 ml Wasser versetzt. Der pH wird mit 0,1 N Natronlauge auf 9 eingestellt. Nach 2,5 Stunden Rühren bei Raumtemperatur wird Essigester dazugegeben und die beiden Phasen getrennt. Die wässrige Phase wird noch dreimal mit Essigester extrahiert und die vereinten Essigesterphasen werden einmal mit Sole gewaschen. Nach Trocknen und Eindampfen erhält man das Rohprodukt, das auf Silicagel mit dem Laufmittel Toluol-Essigester (2:1) chromatographiert wird.
DC (Silicagel): Toluol-Ethylacetat (2:3): $R_f$ = 0,4
IR (Methylenchlorid): 2,94; 5,67; 5,95 $\mu$.

Das Ausgangsmaterial 4-(2—Methyl-tetrazol-5—yl)-thiobuttersäure kann wie folgt hergestellt werden:

1aa) 4-(Tetrazol-5—yl)-buttersäure-ethylester

3,5 g Glutarsäurenitril-monoethylester und 1,78 g Natriumazid werden in Gegenwart von 1,47 g Ammoniumchlorid in 12,5 ml abs. DMF während 25 Stunden bei 125° Badtemperatur gerührt. Nach Abkühlen wird das Reaktionsgemisch mit Wasser verdünnt (150 ml), mit konzentrierter HCl auf pH 3 gestellt und zweimal mit Essigester extrahiert. Nach Trocknung der Essigester-Extrakte und Eindampfen erhält man die Titelverbindung, die durch Umkristallisation aus Ether gereinigt wird. Smp.: 56 - 57°
IR (Methylenchlorid): 3,1; 3,39; 5,78; 6,47; 7,3 $\mu$.

1ab) 4-(2—Methyl-tetrazol-5—yl)-buttersäure-ethylester

14 g 4-(Tetrazol-5'-yl)-buttersäure-ethylester werden in 42 ml Aceton gelöst und nacheinander mit 10,64 ml Triethylamin und 5 ml Methyljodid versetzt. Nach 18 Stunden Rühren unter Argon bei

Rückflusstemperatur wird das Reaktionsgemisch eingedampft. Der Rückstand wird in $CH_2Cl_2$ aufgenommen und mit wässriger $NaHCO_3$-Lösung gewaschen. Nach Trocknen und Eindampfen im Wasserstrahlvakuum wird der Rückstand am Hochvakuum destilliert, wobei man die Titelverbindung isoliert.

Sdp. (0,2 Torr) 116°

IR (Methylenchlorid): 3,39; 5,78; 7,30; 8,51 µ.

1ac) 4-(2—Methyl-tetrazol-5—yl)-buttersäure

10,62 g 4-(2—Methyl-tetrazol-5—yl)-buttersäure-ethylester werden in 270 ml THF mit 80,4 ml 1N NaOH 3h bei Raumtemperatur gerührt. Das THF wird dann am Rotationsverdampfer unter vermindertem Druck grösstenteils abgezogen, die wässrige Phase wird 2 mal mit Essigester gewaschen, und mit 5N HCl auf pH 2 gestellt. Sechsmal wird mit je 60 ml Essigester extrahiert. Die vereinten organischen Phasen werden über Natriumsulfat getrocknet und eingeengt, wobei die Titelverbindung als Feststoff anfällt.

IR (Methylenchlorid); 3,0 bis 3,6; 5,87; 6,7; 7,4 µ

1ad) 4-(2—Methyl-tetrazol-5—yl)-buttersäurechlorid

8,04 g 4-(2—Methyl-tetrazol-5—yl)-buttersäure werden in 180 ml abs. Benzol gelöst und nach Zugabe von 5,47 ml Thionylchlorid und 2 Tropfen DMF wird das Gemisch während 2 Stunden bei 50° gerührt. Nach Abkühlen und Einengen wird das rohe Produkt 30 Minuten am Hochvakuum getrocknet.

IR (Methylenchlorid): 3,4; 5,62; 6,73; 7,17 µ

1ae) 4-(2—Methyl-tetrazol-5—yl)-thiobuttersäure

330 mg 4-(2—Methyl-tetrazol-5—yl)-buttersäurechlorid werden in 0,6 ml abs. Methylenchlorid gelöst und bei 0° tropfenweise zu 2,35 ml einer Lösung von Pyridin und $H_2S$ in Methylenchlorid (100 ml Methylenchlorid, 30 ml Pyridin, 6 g $H_2S$) gegeben. Anschliessend wird 1 Stunde bei 0° unter Stickstoff-Atmosphäre nachgerührt. Das Reaktionsgemisch

wird in Chloroform aufgenommen, mit 2 N $H_2SO_4$ wird die wässrige Phase auf pH 2 angesäuert und zweimal mit Chloroform extrahiert. Die vereinten organischen Phasen werden zweimal mit 2 ml 10 % $NaHCO_3$-Lösung gewaschen. Dann wird mit 2 N $H_2SO_4$ auf pH 3 gestellt, und die Titelverbindung wird durch mehrmaliges Extrahieren mit Chloroform herausextrahiert. Nach Trocknen über Natriumsulfat und Einengen erhält man die Titelverbindung. IR (Methylenchlorid): 3,9; 5,92; 9,35; 9,8 µ.

Das Ausgangsmaterial (3S,4R)-3-(tert.-Butyldimethylsilyloxymethyl)-4-methylsulfonyl-azetidin-2-on kann wie folgt hergestellt werden:

1ba) <u>(3S,5R,6R)-2,2-Dimethyl-6-(tert.-butyl-dimethylsilyloxymethyl)-penam-3-carbonsäuremethylester-1,1-dioxid</u>

Eine Lösung von 23,6 g (85 mMol) (3S,5R,6R)-2,2-Dimethyl-6-hydroxymethyl-penam-3-carbonsäuremethylester-1,1-dioxid in 50 ml Dimethylformamid wird mit 25,5 g (170 mMol) tert.-Butyl-dimethylchlorsilan und 11,5 g (170 mMol) Imidazol bei Raumtemperatur während 45 Minuten gerührt. Dann wird das Lösungsmittel am Hochvakuum abdestilliert und der Rückstand in Aethylacetat aufgenommen. Die Lösung wird mit 1N-Schwefelsäure und dann mit Wasser gewaschen und die wässerigen Lösungen zweimal mit Aethylacetat extrahiert. Die organische Phase wird mit Natriumsulfat getrocknet und am Rotationsverdampfer eingeengt. Das Produkt fällt als kristalline Masse an.

DC Silicagel, Toluol/Aethylacetat (4:1): Rf = 0,56

IR ($CH_2Cl_2$) 3,4; 5,57; 5,65 µm.

1bb) <u>2-[(3S,4R)-3-(tert.-Butyl-dimethylsilyloxymethyl)-4-methylsulfonyl-2-oxo-azetidin-1-yl]-3-methyl-2-butensäuremethylester</u>

Eine Lösung von 202 g (0,51 Mol) (3R,5R,6R)-2,2-Dimethyl-6-(tert.-butyl-dimethylsilyloxymethyl)-penam-3-carbonsäuremethylester-1,1-dioxid in 800 ml Tetrahydrofuran werden mit 9 ml DBU versetzt und während 5 Minuten bei Raumtemperatur gerührt. Dann wurden weitere 95 ml DBU zugegeben und 30 Minuten bei Raumtemperatur gerührt. Anschliessend fügte man unter Kühlung 42,3 ml (0,68 Mol) Methyljodid zu. Nach 3 Stunden Reaktionsdauer wird vom auskristallisierten DBU-Hydrojodid abfiltriert und das Filtrat eingeengt. Der Rückstand wird in Aethylacetat aufgenommen und die Lösung mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und die Lösung zu einem dicken Oel eingeengt.

DC . Silicagel, Toluol/Aethylacetat (4:1); Rf = 0,42

IR ($CH_2Cl_2$) 5,63; 5,81; 6,17 µm.

1bc) <u>(3S,4R)-3-Hydroxymethyl-4-methylsulfonyl-azetidin-2-on und (3S,4R)-3-(tert.Butyl-dimethylsilyloxymethyl)-4-methylsulfonyl-azetidin-2-on</u>

Eine Lösung von 25 g (61,7 mMol) 2-[(3S,4R)-3-(tert.-Butyl-dimethylsilyloxymethyl)-4-methylsulfonyl-2-oxo-azetidin-1-yl]-3-methyl-2-butensäuremethylester in 400 ml Methylenchlorid wird bei -10° mit einem Ozon/Sauerstoffgemisch behandelt. Das Verschwinden des Ausgangsmaterials wird dünnschichtchromatographisch kontrolliert. Nach Beendigung der Reakion werden 30 ml Dimethylsulfid zugegeben und für 3 Stunden bei Raumtemperatur weitergerührt. Die Lösung wird eingeengt und der Rückstand in einem Gemisch von 160 ml Methanol, 24 ml Aethylacetat und 3 ml Wasser aufgenommen und während 40 Minuten auf 70° erwärmt. Das Lösungsmittel wird anschliessend abgezogen und der Rückstand zweimal mit Toluol abgezogen. Das kristallisierende Oel wird in Methylenchlorid aufgenommen und die Kristalle bestehend aus (3S),(4R)-3-Hydroxymethyl-4-methylsulfonyl-azetidin-2-on, durch Filtration isoliert. Das Filtrat wird eingeengt und (3S,4R)-3-(tert.Butyl-dimethylsilyloxymethyl)-4-methylsulfonyl-azetidin-2-on durch Chromatographie an Silicagel mit Toluol/Aethylacetat (3:1) in reiner Form erhalten:

<u>(3S,4R)-3-Hydroxymethyl-4-methylsulfonyl-azetidin-2-on</u>: DC, Silicagel, Toluol/Aethylacetat (1:1); Rf = 0,36, IR: ($CH_2Cl_2$) 2,96; 3,54; 5,61 µm.

(3S,4R)-3-(tert.Butyl-dimethylsilyloxymethyl)-4-methylsulfonyl-azetidin-2-on: DC; Silicagel, Toluol/Aethylacetat (1:1) Rf = 0,06.

Eine Lösung von 14,6 g (81,5 mMol) (3S,4R)-3-Hydroxymethyl-4-methylsulfonyl-azetidin-2-on in 40 ml Dimethylformamid wird mit 24 g (183 mMol) tert.-Butyldimethylchlorsilan und 11 g (163 mMol) Imidazol während 45 Minuten bei Raumtemperatur versetzt. Dann wird das Lösungsmittel am Hochvakuum abgezogen und der Rückstand in Aethylacetat aufgenommen. Die organische Phase wird nacheinander mit 1N-Schwefelsäure, Wasser und Natriumbicarbonatlösung gewaschen. Die wässerigen Phasen werden zweimal mit Aethylacetat extrahiert. Die vereinigten organischen Phasen werden über Natriumsulfat getrocknet und am Rotationsverdampfer eingeengt. Der kristalline Rückstand ist reines (3S,4R)-3-(tert.-Butyl-dimethylsilyloxymethyl)-4-methylsulfonyl-azetidin-2-on.

Beispiel 2: 2-[(3S,4R)-3-tert.Butyl-dimethylsilyloxymethyl-4-[4-(2-methyltetrazol-5-yl)-butyroylthio]-2-oxo-azetidin-1-yl]-2-hydroxy-essigsäure-2-trimethylsilylethylester

Ein Gemisch von 408 mg (3S,4R)-3-(tert.Butyl-dimethylsilyloxymethyl)-4-[4-(2-methyl-tetrazol-5-yl)-butyroylthio]-azetidin-2-on und 450 mg Glyoxylsäure-2-trimethylsilylethylester-ethylhemiketal in 8,7 ml Toluol und 1,7 ml N,N-Dimethylformamid wird mit 4 g Molekularsieb (Typ 4Å 1/16 der Firma Dr. Bender & Dr.Hobein AG, Zürich) versetzt und bei 100° Badtemperatur 8 Stunden unter Schutzgas gerührt. Das Gemisch wird nach Abkühlen über Hyflo filtriert und der Filterrückstand mit Toluol gewaschen. Eindampfen des Filtrats und Trocknung bei 40° im Hochvakuum ergibt das Produkt als gelbes Oel.

DC (Silicagel): Toluol/Ethylacetat (2:3) $R_f$ = 0,55 und 0,47

IR (Methylenchlorid): 2,86; 5,68; 5,8; 6,0 $\mu$.

Beispiel 3: 2-[(3S,4R)-3-tert.-Butyldimethylsilyloxymethyl-4-[4-(2-methyl-tetrazol-5-yl)-butyroylthio]-2-oxo-azetidin-1-yl]-2-triphenylphosphoranylidenessigsäure-2-trimethylsilylethylester

Zu einer Lösung von 573 mg 2-[(3S,4R)-3-tert.-Butyldimethylsilyloxymethyl)-4-[4-(2-methyl-tetrazol-5-yl)-butyroylthio]-2-oxo-azetidin-1-yl]-2-hydroxyessigsäure-2-trimethylsilylethylester in 4,6 ml Tetrahydrofuran werden unter Rühren bei -15° nacheinander 0,14 ml Thionylchlorid und 0,27 ml Triethylamin in 0,5 ml THF innert 10 Minuten zugegeben. Die weisse Suspension wird 1 Stunde bei 0° nachgerührt, und über Hyflo filtriert. Nach Nachwaschen des Rückstands mit Toluol wird am Rotationsverdampfer eingeengt und am Hochvakuum getrocknet. Der Rückstand wird in 4 ml Dioxan gelöst, mit 0,30 g Triphenylphosphin und 0,13 ml 2,6-Lutidin versetzt und während 18 Stunden auf 50° gerührt. Das Gemisch wird über Hyflo filtriert und dieser Rückstand mit Toluol nachgewaschen. Die vereinten Filtrate werden eingedampft und die Chromatographie des Rückstandes an 30 g Silicagel mit Toluol/Ethylacetat (4:1) ergibt das reine Produkt.

DC (Silicagel) Toluol-Ethylacetat (2:3): $R_f$ = 0,52

IR (Methylenchlorid): 5,75; 5,96; 6,25 μ.

Beispiel 4: (5R,6S)-2-[3-(2-Methyltetrazol-5-yl)-propyl]-6-tert.-butyl-dimethylsilyloxymethyl-2-penem-3-carbonsäure-2-trimethylsilylethylester

2,56 g 2-[(3S,4R)-3-tert.Butyl-dimethylsilyloxymethyl-4-[4-(2-methyl-tetrazol-5-yl)-butyroylthio]-2-oxo-azetidin-1-yl]-2-triphenylphosphoranylidenessigsäure-2-trimethylsilylethylester werden in 980 ml Toluol gelöst und unter Stickstoffatmosphäre während 4 Stunden bei Rückflusstemperatur gerührt. Eindampfen des Lösungsmittels und Chromatographie des Rückstandes auf Silicagel mit dem Laufmittel Toluol/Ethylacetat (5:1) ergibt das reine Produkt.

DC (Silicagel): Toluol-Ethylacetat (2:3) $R_f$ = 0,63

IR (Methylenchlorid): 5,66; 5,93; 6,38; 7,7 μ.

Beispiel 5: (5R,6S)-2-[3-(2-Methyl-tetrazol-5-yl)-propyl]-6-hydroxymethyl-2-penem-3-carbonsäure-2-trimethylsilylethylester

94,4 mg (5R,6S)-2-[3-(2-Methyl-tetrazol-5-yl)-propyl]-6-tert.-butyldimethylsilyloxymethyl-2-penem-3-carbonsäure-2-trimethylsilylethylester wird in 2,8 ml abs. THF gelöst und unter Stickstoffatmosphäre nach Abkühlen auf -80° mit 0,1 ml Essigsäure versetzt. Dann gibt man tropfenweise 10,5 ml einer 0,1 M Tetrabutylammoniumfluorid-Lösung in THF dazu, lässt das Gemisch auf Raumtemperatur kommen und rührt 2 Stunden bei dieser Temperatur. Das Lösungsmittelvolumen wird auf 1 ml am Rotationsverdampfer eingeengt, und der Rückstand wird zwischen 14,7 mg Natriumbicarbonat in 5 ml Wasser und 5 ml Essigester aufgetrennt. Die organische Phase wird abgetrennt, die wässrige wird noch 2 mal mit Essigester extrahiert. Die organischen Extrakte werden noch 1 mal mit Wasser gewaschen und über Natriumsulfat getrocknet. Eindampfen im Hochvakuum gibt das Rohprodukt das an 4 g Silicagel mit dem Laufmittel Toluol-Ethylacetat (1:1), chromatographiert wird.
DC (Silicagel) Toluol-Ethylacetat (2:3): $R_f$ = 0,26
IR (Methylenchlorid): 2,77; 5,62; 5,90; 6,33; 7,64 $\mu$.

Beispiel 6: Natrium-(5R,6S)-2-[3-(2-Methyl-tetrazol-5-yl)-propyl]-6-hydroxymethyl-2-penem-3-carboxylat

74,5 mg (5R,6S)-2-[3-(2-Methyl-tetrazol-5-yl)-propyl]-6-hydroxymethyl-2-penem-3-carbonsäure-2-trimethylsilylethylester werden in 1,5 ml abs. THF gelöst und auf -30° gekühlt. Nach Zugabe von 7 ml 0,1 M Tetrabutylammoniumfluoridlösung in THF wird die Temperatur auf 0° erhöht. Nach 30 Minuten Rühren bei dieser Temperatur wird das Gemisch mit 9 ml Essigester und 9 ml Wasser versetzt. Die Lösung wird dann mit 4 N HCl auf pH 3 gestellt. Die Wasserphase wird dann abgetrennt, und die Essigester Lösung wird mit 7,5 ml 0,05 M $NaHCO_3$ Lsg. extrahiert. Nach Abtrennen der wässrigen Schicht wird die organische Lösung noch einmal mit 2 ml 0,0125 M $NaHCO_3$ Lösung extrahiert. Die wässrigen

Phasen werden vereinigt und am Rotationsverdampfer vom verbleibenden Lösungsmittel befreit. Die Lyophilisation ergibt die Titelverbindung.
DC( reversed phase Opti-$UPC_{12}$): Acetonitril-Wasser (1:1) $R_f$ = 0,9
UV (Phosphatpuffer pH 7,4): $\lambda_{max}$ = 306 nm.

Beispiel 7: (3S,4R)-3-(tert.-Butyl-dimethylsilyloxymethyl)--4-[4-(5-methyl-1,3,4-oxadiazol-2-yl)-butyroylthio]-azetidin-2-on

1,76 g 3-(tert.-Butyldimethylsilyloxymethyl)-4-methylsulfonyl-azetidin-2-on und 1,79 g 4-(5-Methyl-1,3,4-oxadiazol-2-yl)-thiobuttersäure werden in 50 ml Methylenchlorid gelöst und bei Raumtemperatur mit 55 ml Wasser und 10,2 ml 1N Natronlauge versetzt. Die Emulsion wird 1,5 h kräftig gerührt, dann wird die organische Phase abgetrennt und die wässrige zweimal mit Methylenchlorid nachextrahiert. Die vereinten organischen Phasen werden einmal mit einer gesättigten wässrigen Natriumbikarbonatlösung und dann mit Sole gewaschen. Nach Abtrennen und Trocknen über Magnesiumsulfat erhält man nach Einengen der Lösung die rohe Titelverbindung, die durch Chromatographie an Silicagel (Laufmittel Toluol-Aethylacetat 1:1) gereinigt wird.
IR (Methylenchlorid): 2,93; 5,65; 5,94; 6,27; 6,37 μ.

Die Ausgangsverbindung 4-(5-Methyl-1,3,4-oxadiazol-2-yl)-thiobuttersäure wird wie folgt hergestellt:

7a) 4-(5-Methyl-1,3,4-oxadiazol-2-yl)-buttersäureethylester

2,95 g 4-(Tetrazol-5-yl)-buttersäure-ethylester (Beispiel 1aa) werden in 32 ml Acetylchlorid bei Rückfluss 4 Stunden erhitzt. Nach Eindampfen im Wasserstrahlvakuum (Rotationsverdampfer) wird der Rückstand mit 20 ml o-Xylol versetzt und 30 Minuten, bis die Gasentwicklung beendet ist, am Rückfluss erhitzt. Nach dem Einengen bei vermindertem Druck wird das Rohprodukt durch Chromatographie an Silicagel gereinigt (Toluol-Essigester 1:1). Die erhaltene Titelverbindung hat folgende IR-Daten:
IR (Methylenchlorid): 5,76; 6,23; 6,33 μ.

7b) 4-(5-Methyl-1,3,4-oxadiazol-2-yl)-buttersäure

8,08 g 4-(5-Methyl-1,3,4-oxadiazol-2-yl)-buttersäureethylester werden in 120 ml Tetrahydrofuran gelöst, mit 45 ml 1N Natronlauge versetzt und während 6 Stunden bei 80° gerührt. Nach Eindampfen des Reaktionsgemisches wird der erhaltene Rückstand in wenig Wasser aufgenommen und mit Methylenchlorid gewaschen. Nach Ansäuern auf pH 3 mit HCl 4 N wird dreimal mit Methylenchlorid extrahiert. Nach Trocknen und Eindampfen erhält man die Titelverbindung.
IR (Methylenchlorid): 3,33; 5,85; 6,27; 6,37 μ.

7c) 4-(5-Methyl-1,3,4-oxadiazol-2-yl)-thiobuttersäure

1,7 g 4-(5-Methyl-1,3,4-oxadiazol-2-yl)-buttersäure werden in 45 ml abs. Methylenchlorid gelöst, zuerst mit 3,07 ml Triethylamin und dann tropfenweise mit einer Lösung von 1,44 ml Chlorameisensäureisobutylester in 15 ml abs. Methylenchlorid versetzt. Nach einer Stunde Rühren bei -10° wird während 2 h bei gleicher Temperatur Schwefelwasserstoff eingeleitet. Der überschüssige $H_2S$ wird dann mit Stickstoff ausgeblasen und das Reaktionsgemisch wird mit 2N $H_2SO_4$ sauer gestellt. Nach Ausschütteln wird die organische Phase abgetrennt und einmal mit Sole gewaschen. Nach Trocknen über Magnesiumsulfat und Eindampfen verbleibt die Titelverbindung als gelbliches Oel.
IR (Methylenchlorid): 3,87; 5,92; 6,29; 6,39 μ.

Beispiel 8: 2-[(3S,4R)-(3-tert.-Butyl-dimethylsilyloxymethyl)-4-[4-(5-methyl-1,3,4-oxadiazol-2-yl)-butyroylthio]-2-oxoazetidin-1-yl]-2-hydroxyessigsäure-2-trimethylsilylethylester

Analog Beispiel 2 werden 1,31 g (3S,4R)-3-(tert.-Butyldimethylsilyloxymethyl)-4-[4-(5-methyl-1,3,4-oxadiazol-2-yl)-butyroylthio]-azetidin-2-on mit 2,17 g Glyoxylsäure-2-trimethylsilylethylester-ethylhemiketal zur Titelverbindung umgesetzt.
IR (Methylenchlorid): 2,86; 5,66; 5,78; 5,93; 6,27; 6,39 µ.

Beispiel 9: 2-[(3S,4R)-3-(tert.-Butyl-dimethylsilyloxymethyl)-4-[4-(5-methyl-1,3,4-oxadiazol-2-yl)-butyroylthio]-2-oxoazetidin-1-yl]-2-triphenylphosphoranylidenessigsäure-2-trimethylsilylethylester

Analog Beispiel 3 werden 0,47 g 2-[(3S,4R)-3-(tert.-Butyl-dimethylsilyloxymethyl)-4-[4-(5-methyl-1,3,4-oxadiazol-2-yl)-butyroylthio]-2-oxo-azetidin-1-yl]-2-hydroxyessigsäure-2-trimethylsilylethylester in die Titelverbindung überführt.
IR (Methylenchlorid): 5,73; 5,93; 6,25 µ.

Beispiel 10: (5R,6S)-2-[3-(5-Methyl-1,3,4-oxadiazol-2-yl)-propyl]-6-(tert.-butyldimethylsilyloxymethyl)-2-penem-3-carbonsäure-2-trimethylsilylethylester

Analog Beispiel 4 werden 0,14 g 2-[(3S,4R)-3-(tert.-Butyl-dimethylsilyloxymethyl)-4-[4-(5-methyl-1,3,4-oxadiazol-2-yl)-butyroyl thio]-2-oxo-azetidin-1-yl]-2-triphenylphosphoranylidenessigsäure-2-trimethylsilylethylester in die Titelverbindung überführt.
IR (Methylenchlorid): 5,62; 5,90; 6,37 µ.

Beispiel 11: (5R,6S)-2-[3-(5-Methyl-1,3,4-oxadiazol-2-yl)-propyl]-6-hydroxymethyl-2-penem-3-carbonsäure-2-trimethylsilylethylester

Analog Beispiel 5 werden 0,25 g (5R,6S)-2-[3-(5-Methyl-1,3,4-oxadiazol-2-yl)-propyl]-6-(tert.-butyldimethylsilyloxymethyl]-2-penem-3-carbonsäure-2-trimethylsilylethylester in die Titelverbindung überführt. IR (Methylenchlorid): 2,77; 5,62; 5,90; 6,37 µ.

Beispiel 12: (5R,6S)-2-[3-(5-Methyl-1,3,4-oxadiazol-2-yl)-propyl]-6-hydroxymethyl-2-penem-3-carbonsäure

176 mg (5R,6S)-2-[3-(5-Methyl-1,3,4-oxadiazol-2-yl)-propyl]-6-hydroxymethyl-2-penem-3-carbonsäure-2-trimethylsilylethylester werden in 6 ml abs. THF gelöst und auf 0° abgekühlt. Die Lösung wird tropfenweise mit 6,15 ml einer 0,1 N Tetrabutylammoniumfluorid Lösung in THF versetzt. Nach 5 min wird erneut 1 ml dieser Lösung zugegeben und nach total 2 h Reaktion wird das Reaktionsgemisch mit 10 ml Wasser verdünnt und am Rotationsverdampfer stark eingeengt. Der Rückstand wird mit wenig Wasser und Ethylacetat sowie 4,1 ml einer 0,1 N HCl-Lösung versetzt. Die wässrige Phase wird abgetrennt und die organische noch zweimal mit Ethylacetat nachextrahiert. Die wässrigen Extrakte geben nach Lyophilisation die Titelverbindung.
UV (Phosphatpuffer pH 7,4) $\lambda_{max}$: 302 nm.

Beispiel 13: (5R,6S)-2-[3-(5-Methyl-1,3,4-oxadiazol-2-yl)-propyl]-6-hydroxymethyl-2-penem-3-carbonsäure-1-ethoxycarbonyloxyethylester

1,2 g Natriumjodid werden in 3,7 ml Aceton gelöst und mit 0,275 ml Ethyl-1-chlorethylcarbonat versetzt. Das Gemisch wird bei Raumtemperatur während 3 h gerührt. Anschliessend wird die Lösung auf 15,0 ml Methylenchlorid getropft und von den ausgefallenen anorganischen Salzen abfiltriert. Die Methylenchloridlösung wird bis auf 2 ml eingeengt und bei 0° zu einer Lösung von 0,325 g (1mMol) (5R,6S)-2-[3-(5-Methyl-1,3,4-oxadiazol-2-yl)-propyl]-6-hydroxymethyl-2-penem-3-carbonsäure in 4 ml Dimethylacetamid gegeben. Dann wird während 3 Stunden bei 0° gerührt, anschliessend mit Ethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Ethylacetat gereinigt. Man erhält die Titelverbindung als weissen Schaum.
IR-Spektrum (Methylenchlorid): Absorptionsbanden bei 5,59 und 5,75 $\mu$.

Beispiel 14: (5R,6S)-2-[3-(5-Methyl-1,3,4-oxadiazol-2-yl)-propyl]-6-hydroxymethyl-2-penem-3-carbonsäure-pivaloyloxymethylester

0,6 g Natriumjodid werden in 2 ml Aceton gelöst und mit 0,15 ml Pivalinsäurechlormethylester versetzt. Das Gemisch wird bei Raumtemperatur während 3 h gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,130 g (0,5 mMol) (5R,6S)-2-[3-(5-Methyl-1,3,4-oxadiazol-2-yl)-propyl]-6-hydroxymethyl-2-penem-3-carbonsäure und 0,07 ml Diisopropylethylamin in 4 ml N,N-Dimethylacetamid bei 0° gegeben. Dann wird während 3 h bei 0° gerührt, anschliessend mit Ethylacetat verdünnt und dreimal mit Wasser gewaschen. Die organische Phase wird über Natriumsulfat getrocknet und am Rotationsverdampfer eingeengt. Das Rohprodukt wird an 10 g Silicagel mit dem Laufmittel Ethylacetat gereinigt. Man erhält die Titelverbindung als weissen Schaum.
IR (Methylenchlorid): 5,59 und 5,78 $\mu$.

Beispiel 15: Trockenampullen oder Vials, enthaltend 0,5 g Natrium-(5R,6S)-2-[3-(2-Methyl-tetrazol-5-yl)-propyl]-6-hydroxymethyl-2-penem-3-carboxylat als Wirksubstanz werden wie folgt hergestellt:

Zusammensetzung (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Patentansprüche für die Vertragsstaaten: BE, CH, LI, DE, FR, GB, IT, LU, NL, SE

1. 2-Heterocyclylniederalkyl-2-penem-Verbindungen der Formel

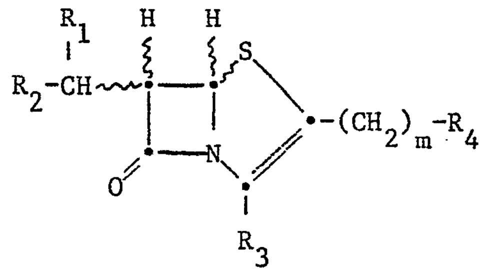
(I),

worin $R_1$ Wasserstoff oder Methyl ist, $R_2$ eine gegebenenfalls geschützte Hydroxyl-Gruppe bedeutet, $R_3$ Carboxyl oder geschütztes Carboxyl $R_3'$ ist, $R_4$ einen über ein Ringkohlenstoffatom an den Rest $-(CH_2)_m-$ gebundenen, ungesättigten monocyclischen Heterocyclyl-Rest darstellt und m eine ganze Zahl von 1 bis 4 ist, Salze von solchen Verbindungen der Formel I, die eine salzbildende Gruppe aufweisen, optische Isomere von Verbindungen der Formel I und Mischungen dieser optischen Isomere.

2. Verbindungen der Formel I gemäss Patentanspruch 1, worin $R_1$ Wasserstoff oder Methyl ist, $R_2$ Hydroxyl oder geschütztes Hydroxyl bedeutet, $R_3$ Carboxyl oder geschütztes Carboxyl $R_3'$, insbesondere unter physiologischen Bedingungen spaltbares verestertes Carboxyl $R_3'$ ist, $R_4$ einen über ein Ringkohlenstoffatom an den Rest $-(CH_2)_m-$ gebundenen 5-gliedrigen oder 6-gliedrigen Heteroaryl-Rest oder partiell gesättigten Heteroarylrest mit 1 bis 4 Ringstickstoffatomen und gegebenenfalls einem zusätzlichen Ringheteroatom der Gruppe Sauerstoff und Schwefel darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Carboxyl, Niederalkoxycarbonyl, gegebenenfalls N-mono- oder N,N-dinieder-

alkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind, und m eine ganze Zahl von 1 bis 4 bedeutet, Salze von Verbindungen der Formel I, die eine salzbildende Gruppe aufweisen, optische Isomere von Verbindungen der Formel I und Mischungen dieser optischen Isomere.

3. Verbindungen der Formel I gemäss Patentanspruch 1, worin $R_4$ einen über ein Ringkohlenstoffatom an den Rest $-(CH_2)_m-$ gebundenen 5-gliedrigen aza-, diaza-, triaza-, tetraza-, oxaza-, oxadiaza-, thiaza-, thiaza-, thiadiaza- oder thiatriaza-cyclischen Rest aromatischen Charakters oder den entsprechenden Dihydro-Rest, oder einen entsprechenden 6-gliedrigen aza-, diaza- und triaza-cyclischen Rest aromatischen Charakters oder den entsprechenden Dihydro- oder Tetrahydro-Rest darstellt, wobei diese Reste unsubstituiert oder, wie in Anspruch 2 definiert, substituiert sind.

4. Verbindungen der Formel I gemäss Patentanspruch 1, worin $R_1$ Wasserstoff oder Methyl ist, $R_2$ Hydroxyl bedeutet, $R_3$ Carboxyl, Niederalkanoyloxymethoxycarbonyl oder 1-Niederalkoxycarbonyloxy-niederalkoxycarbonyl bedeutet, $R_4$ über ein Ringkohlenstoffatom an den Rest $-(CH_2)_m-$ gebundenes, gegebenenfalls durch Niederalkyl, Carboxyniederalkyl, Sulfoniederalkyl oder Diniederalkylaminoniederalkyl substituiertes Tetrazol, wie 1H- oder 2H-Tetrazol-5-yl, gegebenenfalls durch Niederalkyl oder Amino substituiertes Thiadiazolyl, wie 1,3,4-Thiadiazol-2-yl, gegebenenfalls durch Niederalkyl substituiertes Oxadiazolyl, wie 1,3,4-Oxadiazol-2-yl, oder Pyridyl, z.B. 2-Pyridyl, darstellt und m eine ganze Zahl von 2 bis 4 bedeutet, pharmazeutisch verwendbare Salze von solchen Verbindungen der Formel I, die eine salzbildende Gruppe enthalten, optische Isomere, z.B. die (5R,6S)-Isomere, von Verbindungen der Formel I und Mischungen dieser optischen Isomere.

5. Verbindungen der Formel I gemäss Patentanspruch 1, worin $R_4$ 1H-Tetrazol-5-yl, 1-Methyl-1H-tetrazol-5-yl, 1-Carboxymethyl-1H-tetrazol-5-yl, 1-Sulfomethyl-1H-tetrazol-5-yl, 1-(2-Dimethylaminoäthyl)-1H-tetrazol-5-yl, 2-Methyl-2H-tetrazol-5-yl, 1,3,4-Thiadiazol-2-yl, 2-Methyl-1,3,4-thiadiazol-5-yl, 2-Methyl-1,3,4-oxadiazol-5-yl oder 2-Pyridyl bedeutet.

6. (5R,6S)-konfigurierte Peneme der Formel I gemäss Patentanspruch 1, worin $R_1$ Wasserstoff ist, $R_2$ Hydroxyl bedeutet, $R_3$ Carboxyl ist, $R_4$ durch Niederalkyl oder Diniederalkylaminoniederalkyl substituiertes Tetrazol-5-yl, z.B. 1-Methyl-1H-tetrazol-5-yl, 1-(2-Dimethylaminoäthyl)-1H-tetrazol-5-yl oder 2-Methyl-2H-tetrazol-5-yl, oder durch Niederalkyl substituiertes 1,3,4-Oxadiazol-2-yl, z.B. 2-Methyl-1,3,4-oxadiazol-5-yl, darstellt und m 3 ist, und pharmazeutisch verwendbare Salze von Verbindungen der Formel I.

7. (5R,6S)-2-[3-(2-Methyl-2H-tetrazol-5-yl)-propyl]-hydroxymethyl-2-penem-3-carbonsäure und pharmazeutisch verwendbare Salze davon gemäss Patentanspruch 1.

8. (5R,6S)-2-[3-(5-Methyl-1,3,4-oxadiazol-2-yl)-propyl]-6-hydroxymethyl-2-penem-3-carbonsäure und pharmazeutisch verwendbare Salze davon gemäss Patentanspruch 1.

9. Pharmazeutische Präparate enthaltend Verbindungen der Formel I gemäss Patentanspruch 1 oder pharmazeutisch verwendbare Salze von solchen Verbindungen mit salzbildenden Gruppen.

10. Verwendung von Verbindungen der Formel I gemäss Patentanspruch 1 und von pharmazeutisch verwendbaren Salzen von solchen Verbindungen mit salzbildenden Gruppen zur Herstellung von pharmazeutischen Präparaten.

11. Verbindungen der Formel I gemäss Patentanspruch 1 als antibakterielle Mittel.

12. Eine Verbindung gemäss Patentanspruch 1 und ihre pharmazeutisch verwendbaren Salze zur Anwendung in einem Verfahren zur therapeutischen Behandlung des menschlichen und tierischen Körpers.

13. Verfahren zur Herstellung von Verbindungen der Formel

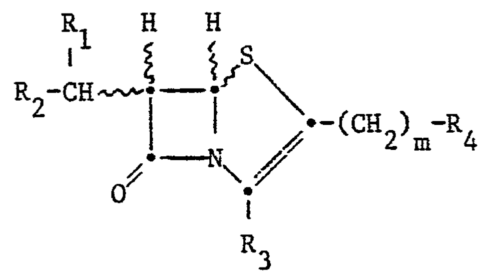

(I),

worin $R_1$ Wasserstoff oder Methyl ist, $R_2$ eine gegebenenfalls geschützte Hydroxyl-Gruppe bedeutet, $R_3$ Carboxyl oder geschütztes Carboxyl $R_3'$ ist, $R_4$ einen über ein Ringkohlenstoffatom an den Rest $-(CH_2)_m-$ gebundenen, ungesättigten, monocyclischen Heterocyclyl-Rest darstellt und m eine ganze Zahl von 1 bis 4 ist, Salze von solchen Verbindungen der Formel I, die eine salzbildende Gruppe aufweisen, optische Isomere von Verbindungen der Formel I und Mischungen dieser optischen Isomere, dadurch gekennzeichnet, dass man

a) eine Ylid-Verbindung der Formel

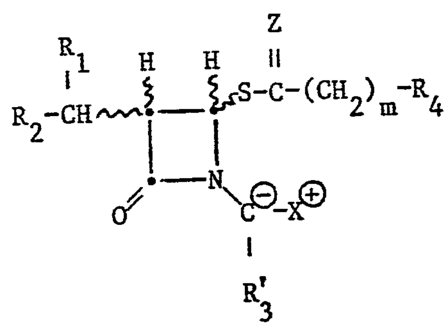

(II),

worin $R_1$, $R_2$, $R_3'$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, Z Sauerstoff oder Schwefel bedeutet und $X^{\oplus}$ entweder eine dreifach substi-

tuierte Phosphoniogruppe oder eine zweifach veresterte Phosphonogruppe zusammen mit einem Kation darstellt, ringschliesst, oder

b) eine Verbindung der Formel

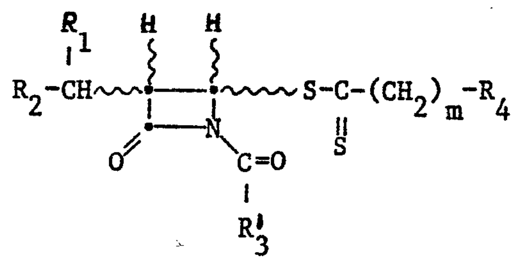

(III) ,

worin $R_1$, $R_2$, $R_3'$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, mit einer organischen Verbindung des dreiwertigen Phosphors behandelt, und wenn erwünscht oder notwendig, in einer erhältlichen Verbindung der Formel I eine geschützte Hydroxylgruppe $R_2$ in die freie Hydroxylgruppe überführt, und/oder, wenn erwünscht, in einer erhältlichen Verbindung der Formel I eine geschützte Carboxylgruppe $R_3'$ in die freie oder in eine andere geschützte Carboxylgruppe $R_3'$ überführt, und/oder, wenn erwünscht, weitere im Rest $R_4$ enthaltende geschützte funktionelle Gruppen in die freien funktionellen Gruppen überführt, und/oder, wenn erwünscht, in einer erhältlichen Verbindung der Formel I einen Rest $R_4$ in einer anderen Rest $R_4$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

14. Die nach dem Verfahren gemäss Anspruch 13 erhältlichen Verbindungen.

Patentansprüche: für den Vertragsstaat AT

1. Verfahren zur Herstellung von Verbindungen der Formel

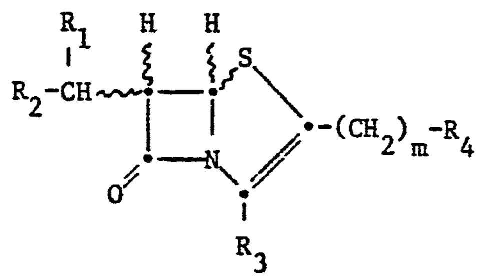 (I),

worin $R_1$ Wasserstoff oder Methyl ist, $R_2$ eine gegebenenfalls geschützte Hydroxyl-Gruppe bedeutet, $R_3$ Carboxyl oder geschütztes Carboxyl $R_3'$ ist, $R_4$ einen über ein Ringkohlenstoffatom an den Rest $-(CH_2)_m-$ gebundenen, ungesättigten, monocyclischen Heterocyclyl-Rest darstellt und m eine ganze Zahl von 1 bis 4 ist, Salze von solchen Verbindungen der Formel I, die eine salzbildende Gruppe aufweisen, optische Isomere von Verbindungen der Formel I und Mischungen dieser optischen Isomere, dadurch gekennzeichnet, dass man

a) eine Ylid-Verbindung der Formel

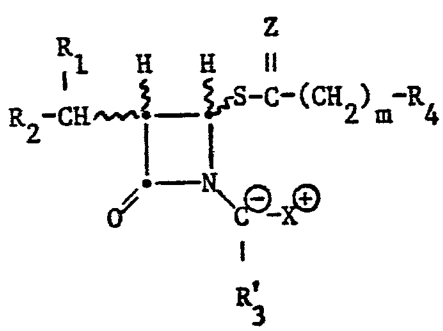 (II),

worin $R_1$, $R_2$, $R_3'$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, Z Sauerstoff oder Schwefel bedeutet und $X^{\oplus}$ entweder eine dreifach substi-

tuierte Phosphoniogruppe oder eine zweifach veresterte Phosphonogruppe zusammen mit einem Kation darstellt, ringschliesst, oder

b) eine Verbindung der Formel

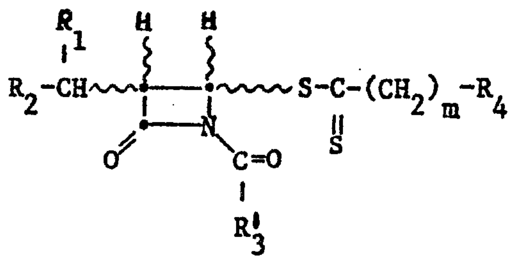

(III) ,

worin $R_1$, $R_2$, $R_3'$, $R_4$ und m die unter Formel I angegebenen Bedeutungen haben, wobei die in den Resten $R_2$ und/oder $R_4$ enthaltenen funktionellen Gruppen gegebenenfalls in geschützter Form vorliegen, mit einer organischen Verbindung des dreiwertigen Phosphors behandelt, und wenn erwünscht oder notwendig, in einer erhältlichen Verbindung der Formel I eine geschützte Hydroxylgruppe $R_2$ in die freie Hydroxylgruppe überführt, und/oder, wenn erwünscht, in einer erhältlichen Verbindung der Formel I eine geschützte Carboxylgruppe $R_3'$ in die freie oder in eine andere geschützte Carboxylgruppe $R_3'$ überführt, und/oder, wenn erwünscht, weitere im Rest $R_4$ enthaltende geschützte funktionelle Gruppen in die freien funktionellen Gruppen überführt, und/oder, wenn erwünscht, in einer erhältlichen Verbindung der Formel I einen Rest $R_4$ in einer anderen Rest $R_4$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

2. Verfahren gemäss Anspruch 1 zur Herstellung von Verbindungen der Formel I, worin $R_1$ Wasserstoff oder Methyl ist, $R_2$ Hydroxyl oder

geschütztes Hydroxyl bedeutet, $R_3$ Carboxyl oder geschütztes Carboxyl $R_3'$, insbesondere unter physiologischen Bedingungen spaltbares verestertes Carboxyl $R_3'$, ist, $R_4$ einen über ein Ringkohlenstoffatom an den Rest $-(CH_2)_m-$ gebundenen 5-gliedrigen oder 6-gliedrigen Heteroaryl-Rest oder partiell gesättigten Heteroaryl-Rest mit 1 bis 4 Ringstickstoffatomen und gegebenenfalls einem zusätzlichen Ringheteroatom der Gruppe Sauerstoff und Schwefel darstellt, wobei diese Reste unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkoxyniederalkyl, Carboxyniederalkyl, Aminoniederalkyl, Diniederalkylaminoniederalkyl, Sulfoniederalkyl, Amino, Niederalkylamino, Diniederalkylamino, Niederalkylenamino, Carboxyl, Niederalkoxycarbonyl, gegebenenfalls N-mono- oder N,N-diniederalkyliertes Carbamoyl, Cyano, Sulfo, Sulfamoyl, gegebenenfalls durch Niederalkyl, Nitro, Niederalkoxy und/oder Halogen substituiertes Phenyl, Cycloalkyl, Nitro, Oxo und/oder Oxido substituiert sind, und m eine ganze Zahl von 1 bis 4 bedeutet, ihrer Salze, ihrer optischen Isomere und Mischungen ihrer optischen Isomere.

3. Verfahren gemäss Anspruch 1 zur Herstellung von Verbindungen der Formel I, worin $R_1$, $R_2$, $R_3$ und m die in Anspruch 2 angegebenen Bedeutungen haben und $R_4$ einen unsubstituierten oder, wie in Anspruch 2 angegeben, substituierten, über ein Ringkohlenstoffatom an den Rest $-(CH_2)_m-$ gebundenen 5-gliedrigen aza-, diaza-, triaza-, tetraza-, oxaza-, oxadiaza-, thiaza-, thiadiaza- oder thiatriaza-cyclischen Rest aromatischen Charakters oder den entsprechenden Dihydro-Rest, oder einen entsprechenden 6-gliedrigen aza-, diaza- und triaza-cyclischen Rest aromatischen Charakters oder den entsprechenden Dihydro- oder Tetrahydro-Rest darstellt.

4. Verfahren gemäss Anspruch 1 zur Herstellung von Verbindungen der Formel I, worin $R_1$ Wasserstoff oder Methyl ist, $R_2$ Hydroxyl bedeutet, $R_3$ Carboxyl, Niederalkanoyloxymethoxycarbonyl oder 1-Niederalkoxycarbonyloxy-niederalkoxycarbonyl, bedeutet, $R_4$ über ein Ringkohlenstoff-

atom an den Rest $-(CH_2)_m-$ gebundenes, gegebenenfalls durch Niederalkyl, Carboxyniederalkyl, Sulfoniederalkyl oder Diniederalkylaminoniederalkyl substituiertes Tetrazol, wie 1H- oder 2H-Tetrazol-5-yl, gegebenenfalls durch Niederalkyl oder Amino substituiertes Thiadiazolyl, wie 1,3,4-Thiadiazol-2-yl, gegebenenfalls durch Niederalkyl substituiertes Oxadiazolyl, wie 1,3,4-Oxadiazol-2-yl, oder Pyridyl, z.B. 2-Pyridyl, darstellt und m eine ganze Zahl von 2 bis 4 bedeutet, ihrer pharmazeutisch verwendbaren Salze, ihrer optischen Isomere, z.B. der (5R,6S)-Isomere, und Mischungen ihrer optischen Isomere.

5. Verfahren gemäss Anspruch 1 zur Herstellung von Verbindungen der Formel I, worin $R_1$, $R_2$, $R_3$ und m die in Anspruch 4 angegebenen Bedeutungen haben und $R_4$ 1H-Tetrazol-5-yl, 1-Methyl-1H-tetrazol-5-yl, 1-Carboxymethyl-1H-tetrazol-5-yl, 1-Sulfomethyl-1H-tetrazol-5-yl, 1-(2-Dimethylaminoäthyl)-1H-tetrazol-5-yl, 2-Methyl-2H-tetrazol-5-yl, 1,3,4-Thiadiazol-2-yl, 2-Methyl-1,3,4-thiadiazol-5-yl, 2-Methyl-1,3,4-oxadiazol-5-yl oder 2-Pyridyl bedeuten.

6. Verfahren gemäss Anspruch 1 zur Herstellung von Verbindungen der Formel I, welche die (5R,6S)-Konfiguration aufweisen.

7. Verfahren gemäss Anspruch 1 zur Herstellung von (5R,6S)-konfigurierten Verbindungen der Formel I, worin $R_1$ Wasserstoff ist, $R_2$ Hydroxyl bedeutet, $R_3$ Carboxyl ist, $R_4$ durch Niederalkyl oder Diniederalkylaminoniederalkyl substituiertes Tetrazol-5-yl, z.B. 1-Methyl-1H-tetrazol-5-yl, 1-(2-Dimethylaminoäthyl)-1H-tetrazol-5-yl oder 2-Methyl-2H-tetrazol-5-yl, oder durch Niederalkyl substituiertes 1,3,4-Oxadiazol-2-yl, z.B. 2-Methyl-1,3,4-oxadiazol-5-yl, darstellt und m 3 ist, und ihrer pharmazeutisch verwendbaren Salze.

8. Verfahren gemäss Anspruch 1 zur Herstellung von (5R,6S)-2-[3-(2-Methyl-2H-tetrazol-5-yl)-propyl]-6-hydroxymethyl-2-penem-3-carbonsäure und pharmazeutisch verwendbarer Salze davon.

9. Verfahren gemäss Anspruch 1 zur Herstellung von (5R,6S)-2-[3-(5-Methyl-1,3,4-oxadiazol-2-yl)-propyl]-6-hydroxymethyl-2-penem-3-carbonsäure und pharmazeutisch verwendbarer Salze davon.

10. Verfahren zur Herstellung von pharmazeutischen Präparaten, dadurch gekennzeichnet, dass man eine nach dem Verfahren gemäss Anspruch 1 erhaltene Verbindung der Formel I oder ein pharmazeutisch verwendbares Salz einer solchen Verbindung mit einem pharmazeutisch verwendbaren Trägermaterial mischt.

0109362

Europäisches Patentamt

# EUROPÄISCHER RECHERCHENBERICHT

Nummer der Anmeldung

EP 83 81 0518

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 950 898 (BRISTOL-MYERS)<br>* Ansprüche 1-5,10,11 *<br>--- | 1,2,9-14 | C 07 D 499/00<br>A 61 K 31/43 //<br>C 07 F 7/18<br>C 07 F 9/65<br>C 07 D 205/08<br>C 07 D 257/04<br>C 07 D 271/10 |
| Y | EP-A-0 002 210 (MERCK)<br>* Ansprüche 1-6,9,10 *<br>--- | 1,2,9-14 | |
| A | EP-A-0 003 960 (CIBA-GEIGY)<br>* Ansprüche 1-11 *<br>----- | 1,2,9-14 | |
| | | | RECHERCHIERTE SACHGEBIETE (Int. Cl. 3) |
| | | | C 07 D 499/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 22-02-1984 | CHOULY J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82